# EUROPEAN PATENT SPECIFICATION

(11) **EP 3 785 652 B1**
(45) Date of publication and mention of the grant of the patent: **08.04.2026**
(21) Application number: 20193507.9
(22) Date of filing: 29.08.2020
(51) Int. Cl.: A61B 18/02, A61B 90/00

(54) **INSTRUMENT FOR PRODUCING TISSUE EFFECTS AT OR NEAR AN ENDOMETRIUM**
INSTRUMENT ZUR ERZEUGUNG VON GEWEBEEFFEKTEN AN ODER IN DER NÄHE EINES ENDOMETRIUMS
INSTRUMENT POUR LA PRODUCTION D'EFFETS DE TISSU SUR OU À PROXIMITÉ D'UN ENDOMÈTRE

(30) Priority: 29.08.2019 US 201962893317 P
(43) Date of publication of application: 03.03.2021
(73) Proprietor: Gyrus ACMI, Inc. d/b/a Olympus Surgical Technologies America, Southborough MA 01772 (US)
(72) Inventor: HOLMAN, Thomas, Princeton, MN 55371 (US); MURDESHWAR, Nikhil M., Maple Grove, MN 55311 (US)
(74) Representative: Noack, Andreas

(56) References cited:
- WO-A1-2016/088120
- KR-A- 20190 045 625
- US-A1- 2009 125 010
- US-A1- 2010 204 688
- US-A1- 2010 262 133
- US-A1- 2012 059 364
- US-A1- 2017 224 379
- US-A1- 2018 133 446
- US-A1- 2019 059 971

## Description

### CLAIM OF PRIORITY

This application claims the benefit of priority to U.S. Provisional Application No. 62/893,317, filed August 29, 2020, titled "INSTRUMENT FOR PRODUCING TISSUE EFFECTS AT OR NEAR AN ENDOMETRIUM".

### BACKGROUND

Abnormal uterine bleeding includes heavy bleeding (also known as menorrhagia), prolonged bleeding, and/or bleeding between monthly periods. Abnormal uterine bleeding can be caused by uterine fibroids, uterine polyps, hormonal issues, and many other causes. Treatment for abnormal uterine bleeding has traditionally included a hysterectomy, which is an invasive surgical procedure. More recently, global endometrial ablation (GEA) has been used for treating abnormal uterine bleeding. GEA is a less invasive procedure that may cause tissue effects in or near the endometrium of the uterus using a suitable ablation technology. US 2009/125010 A1, WO 2016/088120 A1, US 2012/059364 A1, US 2019/059971 A1, US 2010/204688 A1, US 2018/133446 A1, KR 2019 0045625 A, US 2010/262133 A1, and US 2017/224379 A1 disclose related prior art.

### SUMMARY

The invention is defined by independent claim 1. An instrument for producing a tissue effect at or near a uterine wall includes a distal portion configured for receiving a thermal transfer medium. The distal portion being configured to be in fluid communication with at least a portion of a target treatment site, the target treatment site being at or near the uterine wall. The distal portion delivers the thermal transfer medium toward the target treatment site, and thereby produce the tissue effect at or near the uterine wall.

A system for ablating an endometrium of a uterus, the system includes a thermal transfer medium source and an instrument with a nozzle that is configured to be positioned in the uterus to deliver the thermal transfer medium from the thermal transfer medium source directly to the endometrium of the uterus.

An instrument includes an outer shaft that is configured to extend through a vagina and a cervix and into a uterus, a pressure regulator positioned on the outer shaft that is configured to control an inflow rate and an outflow rate of a thermal transfer medium to the uterus, a nozzle positioned in the outer shaft that is configured to be deployed from the outer shaft and positioned in the uterus, and one or more holes in the nozzle that are configured to deliver the thermal transfer medium to the uterus.

A method, which does not form part of the invention, includes (a) delivering a thermal transfer medium toward a uterus, wherein the thermal transfer medium is configured to contact a target treatment site at or near a uterine wall at an inflow rate; (b) exhausting the thermal transfer medium from the target treatment site at an outflow rate; (c) controlling the inflow rate or the outflow rate of the thermal transfer medium so as to distribute the thermal transfer medium so as to produce a tissue effect near the target treatment site; and (d) repeating steps (a) - (c).

A method, which does not form part of the invention, includes (a) delivering a thermal transfer medium to a uterus for a first period of time, wherein the thermal transfer medium is configured to directly contact an endometrium of the uterus; (b) maintaining the thermal transfer medium in the uterus for a second period of time; (c) exhausting the thermal transfer medium from the uterus; and (d) repeating steps (a) - (c).

An instrument includes an outer shaft that is configured to extend through a vagina and a cervix and into a uterus, a nozzle positioned in the outer shaft that is configured to be deployed from the outer shaft and positioned in the uterus, and one or more holes in the nozzle that are configured to deliver a thermal transfer medium to the uterus.

An instrument for producing a tissue effect at or near a uterine wall, the instrument includes a distal portion configured for receiving a thermal transfer medium, wherein the distal portion is configured to be in fluid communication with at least a portion of a target treatment site at or near the uterine wall, and wherein the distal portion is configured to deliver the thermal transfer medium toward the target treatment site and thereby producing the tissue effect at or near the uterine wall. The instrument further includes a first arm connected to the distal portion and extending towards a first fallopian tube, and a first protector operatively coupled to a distal end of the first arm that is configured to block the first fallopian tube.

An instrument includes a nozzle that is configured to be positioned in a uterus, a plurality of holes in the nozzle that are configured to deliver a thermal transfer medium to the uterus, a first arm connected to the nozzle and extending towards a first fallopian tube, a first protector at the distal end of the first arm that is configured to block the first fallopian tube, a second arm connected to the nozzle and extending towards a second fallopian tube, and a second protector at the distal end of the second arm that is configured to block the second fallopian tube.

An instrument includes a nozzle that is configured to be positioned in a uterus, one or more holes in the nozzle that are configured to deliver a thermal transfer medium to the uterus, a first tube that is configured to extend towards a first fallopian tube, and a second tube that is configured to extend towards a second fallopian tube.

A method, which does not form part of the invention, includes inserting an instrument including a nozzle into a uterus, delivering a thermal transfer medium to the uterus through the nozzle, directly contacting an endometrium of the uterus with the thermal transfer fluid, and cooling the endometrium of the uterus with the thermal transfer fluid.

An instrument includes an outer shaft that is configured to extend through a vagina and a cervix and into a uterus, a sealing portion positioned around the outer shaft that is configured to form a seal between the outer shaft and the cervix when it is expanded, a nozzle positioned in the outer shaft that is configured to be deployed from the outer shaft and positioned in the uterus, and one or more holes in the nozzle that are configured to deliver a thermal transfer medium to the uterus.

### BRIEF DESCRIPTION OF THE DRAWINGS

FIG. 1A is a perspective view of a first example of an instrument, not being part of the invention.
FIG. 1B is a schematic view of the first example of the instrument, a thermal transfer medium source, and an exhaust system.
FIG. 1C is a schematic view of a distal end of the first example of the instrument positioned in a uterus.
FIG. 2 is a schematic view of a first thermal transfer medium being delivered to the uterus through a nozzle of the first example of the instrument.
FIG. 3 is a graph showing a time versus temperature profile.
FIG. 4 is a schematic view of a second thermal transfer medium being delivered to the uterus through the nozzle of the first example of the instrument.
FIG. 5 is a schematic view of a third thermal transfer medium being delivered to the uterus through the nozzle of the first example of the instrument.
FIG. 6A is a schematic view of a second example of an instrument, a fluid source, a thermal transfer medium source, and an exhaust system.
FIG. 6B is a schematic view of a distal end of the second example of the instrument positioned in the uterus.
FIG. 7A is a schematic view of a third example of an instrument, which does not form part of the invention, a thermal transfer medium source, and an exhaust system.
FIG. 7B is a schematic view of a distal end of the third example of the instrument positioned in the uterus.
FIG. 8A is a perspective view of a fourth example of an instrument, which does not form part of the invention.
FIG. 8B is a schematic view of the fourth example of the instrument, a fluid source, a thermal transfer medium source, and an exhaust system.
FIG. 8C is a schematic view of a distal end of the fourth example of the instrument positioned in the uterus.
FIG. 9A is a schematic view of a fifth example of an instrument, which does not form part of the invention, a thermal transfer medium source, and an exhaust system.
FIG. 9B is a schematic view of a distal end of the fifth example of the instrument, which does not form part of the invention, positioned in the uterus.
FIG. 10A is a schematic view of a sixth example of an instrument, a thermal transfer medium source, and an exhaust system.
FIG. 10B is a schematic view of a distal end of the sixth example of the instrument positioned in the uterus with unfurl members in a furled state.
FIG. 10C is a schematic view of a distal end of the sixth example of the instrument positioned in the uterus with unfurl members in an unfurled state.
FIG. 11A is a schematic view of a seventh example of an instrument, which does not form part of the invention, a thermal transfer medium source, and an exhaust system.
FIG. 11B is a schematic view of a distal end of the seventh example of the instrument positioned in the uterus with pledgets in an unexpanded state.
FIG. 11C is a schematic view of a distal end of the seventh example of the instrument positioned in the uterus with pledgets in an expanded state.
FIG. 12A is a schematic view of an eighth example of an instrument, which does not form part of the invention, a thermal transfer medium source, and an exhaust system.
FIG. 12B is a schematic view of a distal end of the eighth example of the instrument positioned in the uterus.
FIG. 13A is a schematic view of a ninth example of an instrument, which does not form part of the invention, a fluid source, a thermal transfer medium source, and an exhaust system.
FIG. 13B is a schematic view of a distal end of the ninth example of the instrument positioned in the uterus.
FIG. 14A is a schematic view of a tenth example of an instrument, which does not form part of the invention, a suction mechanism, a thermal transfer medium source, and an exhaust system.
FIG. 14B is a schematic view of a distal end of the tenth example of the instrument positioned in the uterus.
FIG. 15A is a schematic view of an eleventh example of an instrument, which does not form part of the invention, a vacuum source, a thermal transfer medium source, and an exhaust system.
FIG. 15B is a schematic view of a distal end of the eleventh example of the instrument positioned in the uterus.
FIG. 16A is a schematic view of a twelfth example of an instrument, which does not form part of the invention, a foam source, a thermal transfer medium source, and an exhaust system.
FIG. 16B is a schematic view of a distal end of the twelfth example of the instrument positioned in the uterus.
FIG. 17A is a schematic view of a thirteenth example of an instrument, which does not form part of the invention, a thermal transfer medium source, and an exhaust system.
FIG. 17B is a schematic view of a distal end of the thirteenth example of the instrument positioned in the uterus.
FIG. 18A is a schematic view of a fourteenth example of an instrument, which does not form part of the invention, a thermal transfer medium source, and an exhaust system.
FIG. 18B is a schematic view of a distal end of the fourteenth example of the instrument positioned in the uterus.
FIG. 19A is a schematic view of a fifteenth example of an instrument, which does not form part of the invention, a thermal transfer medium source, and an exhaust system.
FIG. 19B is a schematic view of a distal end of the fifteenth example of the instrument positioned in the uterus.
FIG. 19C is a schematic view of the distal end of an embodiment of the instrument that includes seals positioned in the uterus.
FIG. 20A is a schematic view of a sixteenth example of an instrument, which does not form part of the invention, a thermal transfer medium source, and an exhaust system.
FIG. 20B is a schematic view of a distal end of the sixteenth example of the instrument positioned in the uterus.

### DETAILED DESCRIPTION

An instrument is disclosed for producing a tissue effect at or near a uterine wall. The instrument includes a distal portion configured for receiving a thermal transfer medium. The distal portion is configured to be in fluid communication with at least a portion of a target treatment site. The target treatment site is at or near the uterine wall. The distal portion of the instrument delivers the thermal transfer medium toward the target treatment site and thereby produces the tissue effect at or near the uterine wall.

The instrument is disclosed for delivering a thermal transfer medium to a uterus to produce a tissue effect (e.g., ablate) at or near the endometrium of the uterus. The instrument can include an outer shaft, an inner shaft with a nozzle at a distal end of the inner shaft, and one or more holes extending through the nozzle. The outer shaft of the instrument can be inserted through the vagina and the cervix and into the uterus. The inner shaft can then be deployed so that the nozzle is positioned in the uterus. A thermal transfer medium can be delivered to the uterus through the one or more holes in the nozzle.

The thermal transfer medium can include a fluid in a cryogenic state, a cryogenic supercritical fluid, or thermal transfer particles and a transport medium. The flow of thermal transfer medium into the uterus can be cycled to control a time versus temperature profile of the thermal transfer medium in the uterus. The instrument can include a temperature sensor and/or a pressure sensor to sense the temperature and/or pressure of the thermal transfer medium in the uterus. The temperature and pressure readings from the temperature sensor and pressure sensor can be used to control the cycling of the thermal transfer medium in the uterus.

Further, the instrument can optionally include arms with protectors at the distal ends of the arms. The arms can extend towards the fallopian tubes and the protectors can cover the openings to the fallopian tubes to prevent the thermal transfer medium in the uterus from flowing through the fallopian tubes. Additionally, the instrument can include tubes that extend towards the openings of the fallopian tubes to effect a change at the openings to the fallopian tubes to prevent the thermal transfer medium from flowing through the fallopian tubes. Further, the instrument can include more than one nozzle or a nozzle with a particular shape to control where the thermal transfer medium is flowing in the uterus.

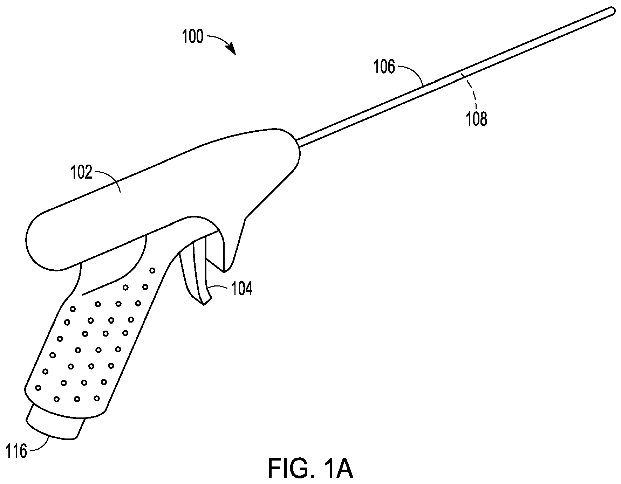

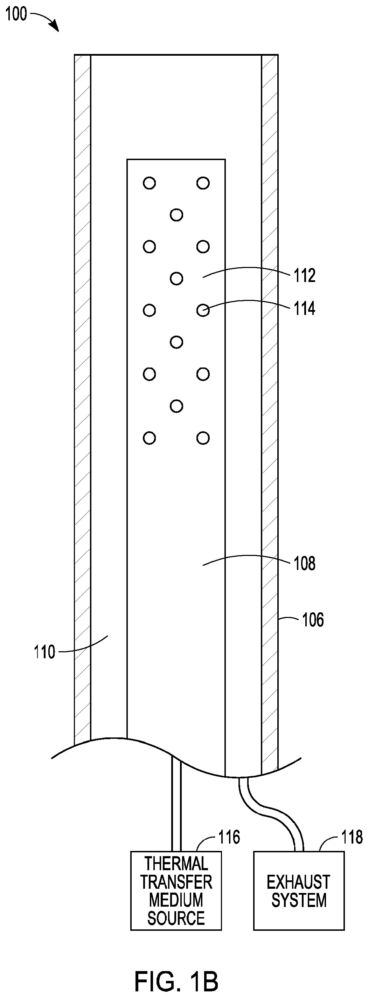

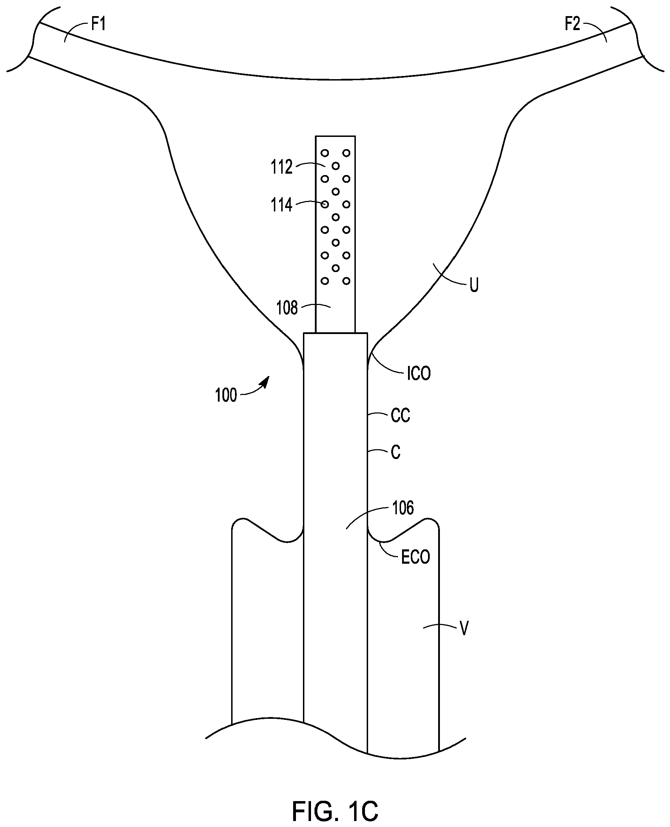

FIGS. 1A-1C show instrument 100 and will be discussed together. FIG. 1A is a perspective view of instrument 100. FIG. 1B is a schematic view of instrument 100. FIG. 1C is a schematic view of a distal end of instrument 100 positioned in uterus U. Instrument 100 includes hand piece 102, trigger 104, outer shaft 106, inner shaft 108, gap 110, nozzle 112, and one or more holes 114. FIGS. 1A-1B also show thermal transfer medium source 116 connected to instrument 100. FIG. 1B further shows exhaust system 118 connected to instrument 100. FIG. 1C also shows vagina V, cervix C (including external cervical os ECO, cervical canal CC, and internal cervical os ICO), uterus U, first fallopian tube F1, and second fallopian tube F2.

Instrument 100 includes hand piece 102. Hand piece 102 forms a body portion of instrument 100 that is configured to be held by a user. Hand piece 102 can be ergonomically designed with a portion that is configured to be gripped by the user. Trigger 104 is connected to hand piece 102 and is positioned to be pulled by a user when the user is gripping hand piece 102. Outer shaft 106 and inner shaft 108 extend away from hand piece 102. In the embodiment shown in FIGS. 1A-1C, outer shaft 106 and inner shaft 108 have a cylindrical shape, but outer shaft 106 and outer shaft 108 can have any suitable shape in alternate embodiments. Outer shaft 106 and inner shaft 108 each include a bore extending from a proximal end to a distal end. Inner shaft 108 is positioned in and extends through the bore of outer shaft 106. Gap 110 is formed between an inner surface of outer shaft 106 and an outer surface of inner shaft 108. A distal end of inner shaft 108 forms nozzle 112. Nozzle 112 includes one or more holes 114 extending from an interior to an exterior of nozzle 112. Plurality of holes 114 can be positioned on nozzle 112 in any suitable pattern. Further, one or more holes 114 can have any suitable shape and size. Additionally, one or more holes 114 can include any number of holes.

Instrument 100 is configured to be inserted through vagina V and cervix C into uterus U. Vagina V is a canal that extends from the vulva (the external female organs) to cervix C. Cervix C forms the lower part of uterus U and includes external cervical os ECO, cervical canal CC, and internal cervical os ICO. External cervical os ECO is the opening between cervix C and vagina C. Internal cervical os ICO is the opening between cervix C and uterus U. Cervical canal CC extends from external cervical os ECO to internal cervical os ICO. First fallopian tube F1 and second fallopian tube F2 connect to a top part of uterus U. First fallopian tube F1 and second fallopian tube F2 connect to the first ovary and second ovary, respectively, and deliver eggs from the first ovary and the second ovary to uterus U.

Uterus U includes three layers: the endometrium, the myometrium, and the perimetrium. The endometrium is the innermost layer and includes a basal layer and a functional layer. The functional layer thickens and sheds during each menstrual cycle. The myometrium is the middle layer and mostly consists of muscle. The perimetrium is the outermost layer that covers the outer surface of uterus U.

As shown in FIG. 1B, when instrument 100 is in a stowed position, nozzle 112 is positioned in the distal end of outer shaft 106. The distal end of outer shaft 106 is configured to be inserted through vagina V and cervix C and into uterus U when nozzle 112 is stowed in instrument 100. After the distal end of outer shaft 106 is positioned in uterus U, nozzle 112 can be deployed from outer shaft 106 so that it is positioned in uterus U, as shown in FIG. 1C. Nozzle 112 is deployed by pulling trigger 104 on hand piece 102, which actuates a lever in instrument 100 to deploy nozzle 112. Alternatively, any suitable mechanism can be used to deploy nozzle 112 from outer shaft 106 in alternate embodiments. When nozzle 112 is deployed in uterus U, a thermal transfer medium can be delivered to uterus U through one or more holes 114 in nozzle 112.

In the embodiment shown in FIG. 1A, thermal transfer medium source 116 is a canister that is inserted into hand piece 102. Thermal transfer medium source 116 is configured to contain and dispense a thermal transfer medium. In alternate embodiments, thermal transfer medium source 116 can be any suitable container that is capable of containing and dispensing a thermal transfer medium source. Further, thermal transfer medium source 116 can be integrally formed with instrument 100 in alternate embodiments. As shown in FIG. 1B, thermal transfer medium source 116 is fluidly coupled to inner shaft 108 of instrument 100. When nozzle 112 is deployed in uterus U, thermal transfer medium source 116 can dispense a thermal transfer medium that will flow through inner shaft 108, nozzle 112, and out through one or more holes 114 into uterus U.

As also shown in FIG. 1B, outer shaft 108 is fluidly coupled to exhaust system 118. Exhaust system 118 can be any suitable mechanism that is capable of evacuating the thermal transfer medium from uterus U. For example, exhaust system 118 could be a vacuum that is capable of sucking the thermal transfer medium out of uterus U. When exhaust system 118 is activated, the thermal transfer medium will flow from uterus U, through gap 110 between inner shaft 108 and outer shaft 106, and into exhaust system 118.

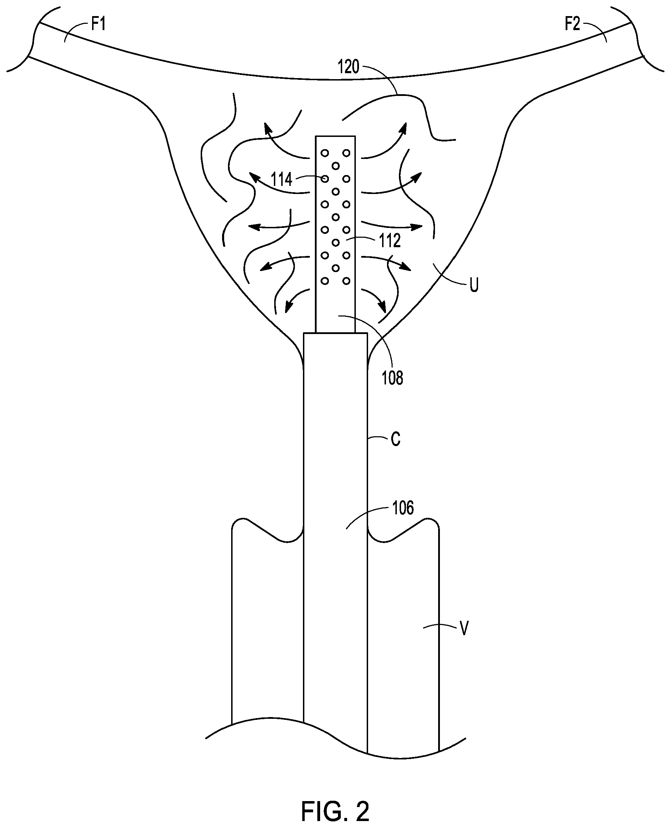

Instrument 100 is configured to deliver a thermal transfer medium to uterus U to ablate the endometrium of uterus U. The thermal transfer medium can include a cryogenic medium that is configured to freeze the endometrium to destroy the endometrial tissue. When the endometrial tissue is destroyed, scar tissue will grow in its place. As a result, the endometrial tissue will not grow and shed during the menstrual cycle to prevent bleeding. The thermal transfer mediums that can be used to ablate the endometrium and the manner of ablating the endometrium are discussed in greater detail below with respect to FIGS. 2-5.

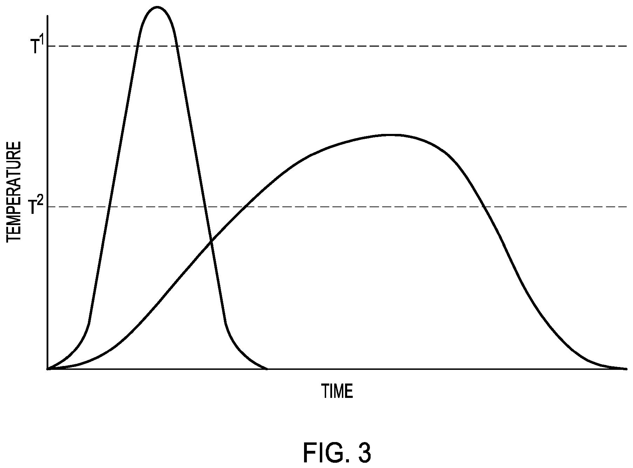

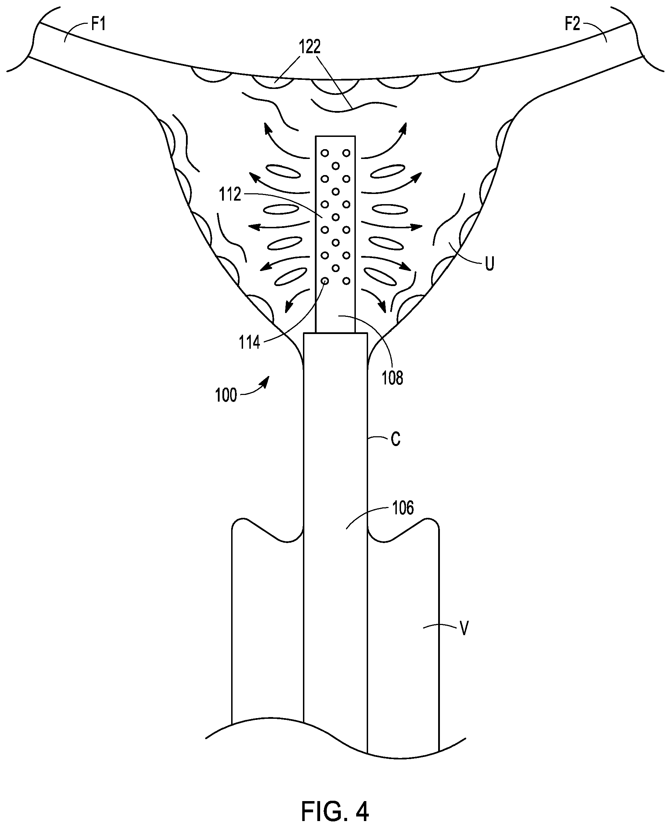

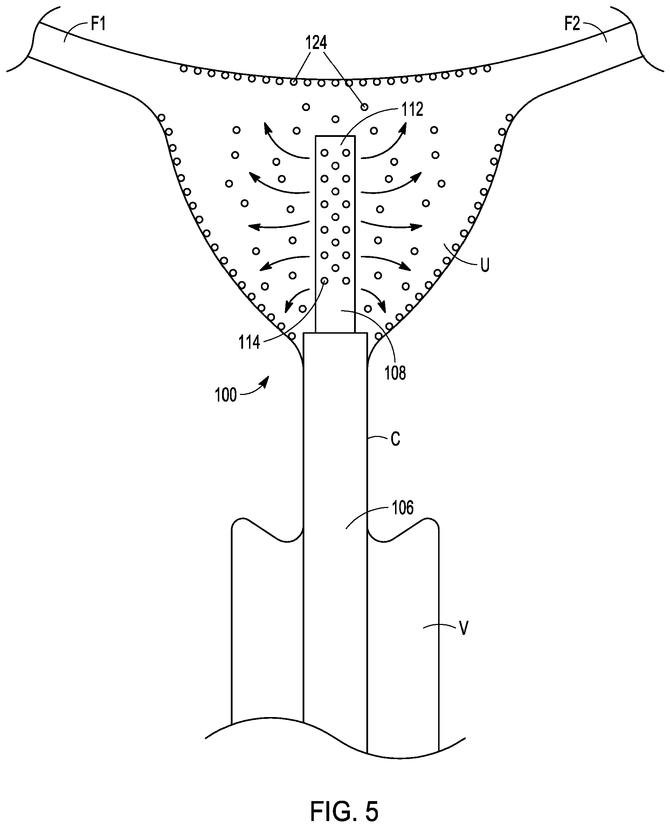

FIG. 2 is a schematic view of a first thermal transfer medium being delivered to uterus U through nozzle 112 of instrument 100. FIG. 3 is a graph showing a time versus temperature profile. FIG. 4 is a schematic view of a second thermal transfer medium being delivered to uterus U through nozzle 112 of instrument 100. FIG. 5 is a schematic view of a third thermal transfer medium being delivered to uterus U through nozzle 112 of instrument 100. FIGS. 2 and 4-5 show instrument 100, which includes outer shaft 106, inner shaft 108, nozzle 112, and one or more holes 114. FIGS. 2 and 4-5 also show vagina V, cervix C, uterus U, first fallopian tube F1, and second fallopian tube F2. FIG. 2 further shows fluid 120. FIG. 4 further shows cryogenic supercritical fluid 122. FIG. 5 further shows thermal transfer particles 124.

Instrument 100 has the same structure and design as discussed above in reference to FIGS. 1A-1C. Outer shaft 106 of instrument 100 is configured to be inserted through vagina V and cervix C and into uterus U. Inner shaft 108 is configured to be deployed from outer shaft 106 when a distal end of outer shaft 106 is positioned in uterus U. Nozzle 112 forms a distal end of inner shaft 108 and includes one or more holes 114 that are configured to deliver a thermal transfer medium to uterus U.

The thermal transfer medium that is delivered to uterus U is configured to ablate the endometrium of uterus U. The thermal transfer medium can include a cryogenic fluid that is configured to cool and freeze the endometrium of uterus U.

The thermal transfer medium can take a number of different forms. Three examples are provided below with reference to FIGS. 2-4. These examples are not intended to be limiting.

FIG. 2 shows a first thermal transfer medium in the form of fluid 120 in a cryogenic state. Fluid 120 is configured to convectively cool the endometrium of uterus U. For example, fluid120 can include nitrous oxide, carbon dioxide, liquid oxygen, and helium.

Thermal transfer medium source 116 (shown in FIGS. 1A-1B) can contain fluid 120 in a liquefied state. As fluid 120 is dispensed from thermal transfer medium source 116, it will reach its boiling point and transition to a gas that flows through inner shaft 106 and nozzle 112 and out through one or more holes 114 into uterus U. Fluid 120 will be in a cryogenic state as it flows into uterus U. Fluid 120 can then contact the endometrium of uterus U to convectively cool the endometrium.

One or more holes 114 can be configured to control the flow of fluid 120 through nozzle 112 into uterus U. One or more holes 114 can have any suitable shape and size to control the flow of fluid 120 through nozzle 112. Further, nozzle 112 can include any suitable number of holes to control the flow of fluid 120 through nozzle 112. Additionally, one or more holes 114 can be positioned on nozzle 112 in any suitable manner to control the flow of fluid 120. Specifically, one or more holes 114 can be positioned on nozzle 112 to direct fluid 120 to a particular location on uterus U. Further, one or more holes 114 can be positioned on nozzle 112 to create a vortex of gas flow in uterus U to circulate fluid 120 within uterus U.

Fluid 120 can also be cycled into uterus U to control the flow of fluid 120 through uterus U. For example, fluid 120 can be dispensed into uterus U for a first period of time at a first flow rate, fluid 120 can be held in uterus U for a second period of time, and then fluid 120 can be exhausted from uterus U at a second flow rate. The first period of time and the second period of time can be predetermined periods of time or they can be determined based on a temperature and/or a pressure of uterus U. The first flow rate and the second flow rate can be predetermined flow rates or they can be determined based on a temperature and/or a pressure of uterus U. This cycle can be repeated as needed until the endometrium is fully ablated.

Curve A on the graph shown in FIG. 3 shows a time versus temperature profile if a thermal transfer medium, such as fluid 120, is fully dispensed into uterus U at one time. As shown by curve A, the temperature will drop very quickly to a very low temperature (over T₂, which can, for example, be about -100 degrees Celsius, in the graph shown in FIG. 3) but the temperature will also raise very quickly after it drops. When the thermal transfer medium, such as fluid 120, is fully dispensed into uterus U at one time it is hard to control the flow of the thermal transfer medium in uterus U and some areas of uterus U may not be treated.

Curve B on the graph shown in FIG. 3 shows a time versus temperature profile if the thermal transfer medium, such as fluid 120, is cycled into uterus U over time. As shown by curve B, the temperature can drop (between T₁ and T₂, which can, for example, be between about -50 degrees Celsius and -100 degrees Celsius, in the graph shown in FIG. 3) over a longer period of time as the time versus temperature profile can be more closely controlled. Controlling the time versus temperature profile, as shown by curve B, offers superior treatment outcomes, as the flow of the thermal transfer medium in uterus U can be more closely controlled. Controlling the flow of the thermal transfer medium in uterus U can ensure that the thermal transfer medium is treating the entirety of the endometrium of the uterus U, which will improve the depth and coverage of ablation of the endometrium of uterus U. The ideal time versus temperature profile can vary based on the thermal transfer medium that is being used.

FIG. 4 shows a second thermal transfer medium in the form of cryogenic supercritical fluid 122. A supercritical fluid is a fluid at a temperature and pressure above its critical point where a clear transition between liquid and gas does not exist. Cryogenic supercritical fluid 122 is configured to convectively cool the endometrium of uterus U. For example, supercritical fluid 122 can include liquid nitrogen, water, and carbon dioxide.

Thermal transfer medium source 116 (shown in FIGS. 1A-1B) can contain super critical fluid 122 in its supercritical fluid state. As cryogenic supercritical fluid 122 is dispensed from thermal transfer medium source 116, it will flow through inner shaft 106 and nozzle 112 and out through one or more holes 114 to uterus U in its supercritical fluid state. Cryogenic supercritical fluid 122 can then contact the endometrium of uterus U. Cryogenic supercritical fluid 122 can boil as it contacts the endometrium of uterus U to convectively cool the endometrium.

One or more holes 114 can be configured to control the flow of cryogenic supercritical fluid 122 through nozzle 112 into uterus U. One or more holes 114 can have any suitable shape and size to control the flow of cryogenic supercritical fluid 122 through nozzle 112. Further, nozzle 112 can include any suitable number of holes to control the flow of cryogenic supercritical fluid 122 through nozzle 112. Additionally, one or more holes 114 can be positioned on nozzle 112 in any suitable manner to control the flow of cryogenic supercritical fluid 122. Specifically, one or more holes 114 can be positioned on nozzle 112 to direct supercritical fluid 122 to particular locations on uterus U.

Cryogenic supercritical fluid 122 can be selected for its time release constant. Specifically, cryogenic supercritical fluid 122 can be selected so that it boils upon contact with the endometrium of uterus U, rather than reaching its boiling point prior to contacting the endometrium of uterus U. Cryogenic supercritical fluids 122 can reach cold temperatures very quickly, providing for quicker treatment. Further, cryogenic supercritical fluid 122 can be released in cycles to control the time versus temperature profile of the cryogenic supercritical fluid 122, as discussed above in reference to FIG. 3.

FIG. 5 shows a third thermal transfer medium that includes thermal transfer particles 124 and a transport medium. Thermal transfer particles 124 are configured to conductively cool the endometrium of uterus U and the transport medium is configured to convectively cool the endometrium of uterus U. For example, thermal transfer particles 124 can be sponge, gelatin, metallic balls (such as BBs), and metal particles.

Thermal transfer medium source 116 (shown in FIGS. 1A-1B) can contain thermal transfer particles 124 and a transport medium. The transport medium can be a fluid in a cryogenic state. As thermal transfer particles 124 are dispensed from thermal transfer medium source 116, they will flow through inner shaft 106 and nozzle 112 and out through one or more holes 114 to uterus U with the transport medium. Thermal transfer particles 124 can then contact the endometrium of uterus U. As thermal transfer particles 124 contact the endometrium of uterus U, thermal transfer particles 124 can conductively cool the endometrium and the transport medium can transfer from thermal transfer particles 124 to the endometrium of uterus U to convectively cool the endometrium. This allows the endometrium to be cooled in the spots where thermal transfer particles 124 contact the endometrium but also in areas surrounding the thermal transfer particles 124.

One or more holes 114 can be configured to control the flow of thermal transfer particles 124 through nozzle 112 into uterus U. One or more holes 114 can have any suitable shape and size to control the flow of thermal transfer particles 124 through nozzle 112. Further, nozzle 112 can include any suitable number of holes to control the flow of thermal transfer particles 124 through nozzle 112. Additionally, one or more holes 114 can be positioned on nozzle 112 in any suitable manner to control the flow of thermal transfer particles 124. Specifically, one or more holes 114 can be positioned on nozzle 112 to direct thermal transfer particles 124 to particular locations on uterus U.

Instrument 100 allows the endometrium of uterus U to be directly treated, as the thermal transfer medium (including any of fluid 120, cryogenic supercritical fluid 122, and/or thermal transfer particles 124 with the transport medium) comes into direct contact with the endometrium. In instances of uterine abnormality, such as a septate uterus having a wall of muscle coming down through a center of the uterus, instrument 100 can still effectively treat the entire endometrium of uterus U. Traditional ablation devices that utilize a balloon cannot properly treat some uteruses with uterine abnormalities, as the balloon cannot effectively cover the entirely of the endometrium. As instrument 100 directs the flow of thermal transfer medium directly to the endometrium of uterus U, the entirety of uterus U can be treated even in cases of uterine abnormalities.

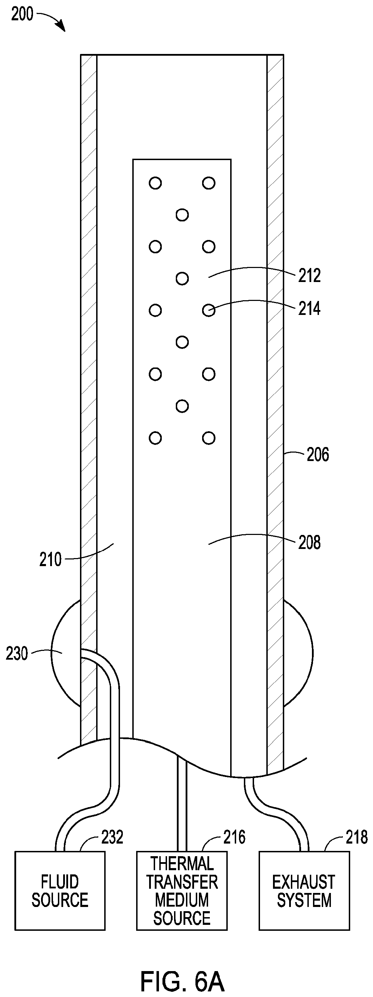

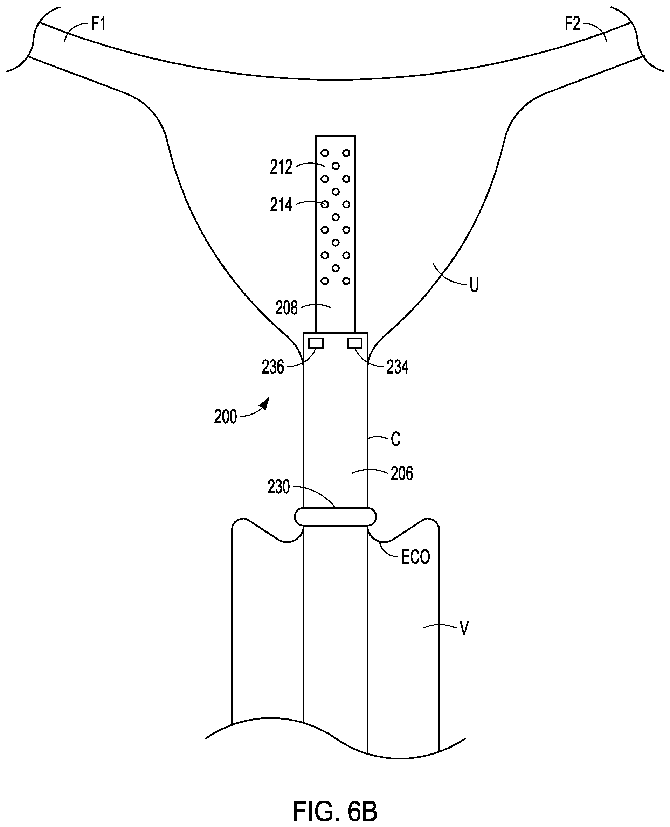

FIG. 6A is a schematic view of instrument 200, fluid source 232, thermal transfer medium source 216, and exhaust system 218. FIG. 6B is a schematic view of a distal end of instrument 200 with sealing portion 230 positioned in uterus U. Instrument 200 includes outer shaft 206, inner shaft 208, gap 210, nozzle 212, and one or more holes 214. FIG. 6A also shows thermal transfer medium source 216 and exhaust system 218. FIGS. 6A-6B further show sealing portion 230 of instrument 200, FIG. 6A shows fluid source 232, and FIG. 6B shows temperature sensor 234 and pressure sensor 236. FIG. 6B also shows vagina V, cervix C, external cervical os ECO, uterus U, first fallopian tube F1, and second fallopian tube F2.

Instrument 200 has generally the same structure and design as instrument 100 described above in reference to FIGS. 1A-1C, however instrument 200 also includes sealing portion 230, fluid source 232, temperature sensor 234, and pressure sensor 236. Sealing portion 230, fluid source 232, temperature sensor 234, and pressure sensor 236 are described here with respect to instrument 200, but can also be included on any of the embodiments of an instrument descried herewith, including instruments 100, 300, 400, 500, 600, 700, 800, 900, 1000, 1100, 1200, 1300, 1400, 1500, and 1600.

Instrument 200 can include a hand piece and a trigger, not shown in FIGS. 6A-6B. Outer shaft 206 of instrument 200 is configured to be inserted through vagina V and cervix C and into uterus U. Inner shaft 208 is configured to be deployed from outer shaft 206 when a distal end of outer shaft 206 is positioned in uterus U. Gap 210 is formed between an outer surface of inner shaft 208 and an inner surface of outer shaft 206. Nozzle 212 forms a distal end of inner shaft 208 and includes one or more holes 214 that are configured to deliver a thermal transfer medium to uterus U. Any suitable thermal transfer medium, such as those discussed above in reference to FIGS. 2-5, can be delivered to uterus U.

Thermal transfer medium source 216 is fluidly coupled to inner shaft 208 and is configured to deliver a thermal transfer medium to uterus U through inner shaft 208, nozzle 212, and one or more holes 214. Exhaust system 218 is fluidly coupled to outer shaft 206 and is configured to exhaust the thermal transfer medium from uterus U through outer shaft 206.

Instrument 200 further includes sealing portion 230. Sealing portion 230 is a member positioned around outer shaft 206 of instrument 200 adjacent to a distal end of outer shaft 206. As shown in FIG. 6B, sealing portion 230 can be positioned adjacent to external cervical os ECO when the distal end of outer shaft 206 is positioned in uterus U. As shown in FIG. 6A, sealing portion 230 is fluidly coupled to fluid source 232. Fluid source 232 can provide a fluid to sealing portion 230 to expand sealing portion 230 to form a seal between outer shaft 206 and cervix C, as shown in FIG. 6B. The seal formed by sealing portion 230 between outer shaft 206 and cervix C contains the thermal transfer medium in uterus U.

Instrument 200 also includes temperature sensor 234 and pressure sensor 236. Temperature sensor 234 and pressure sensor 236 are positioned on the distal end of outer shaft 206 in the embodiment shown in FIGS. 6A-6B to sense the temperature and pressure in uterus U. In alternate embodiments, temperature sensor 234 and pressure sensor 236 can be positioned on nozzle 212 to sense the temperature and pressure in uterus U. In further alternate embodiments, temperature sensor 234 and pressure sensor 236 can be positioned anywhere on outer shaft 206 to sense the temperature and pressure in vagina V or cervix C. Further, instrument 200 can include any number of temperature sensors and pressure sensors.

Temperature sensor 234 and pressure sensor 236 shown in FIG. 6B can be used to determine the temperature and pressure in uterus U as a thermal transfer medium is being dispensed into uterus U through nozzle 212. As discussed above in reference to FIGS. 2-5, the thermal transfer medium can be cycled into uterus U to control the time to temperature profile of the thermal transfer medium in uterus U. Temperature sensor 234 and pressure sensor 236 can sense the temperature and pressure of the thermal transfer medium in uterus U to indicate to a controller when to dispense the thermal transfer medium from thermal transfer medium source 216 and when to exhaust the thermal transfer medium from uterus U using exhaust system 218.

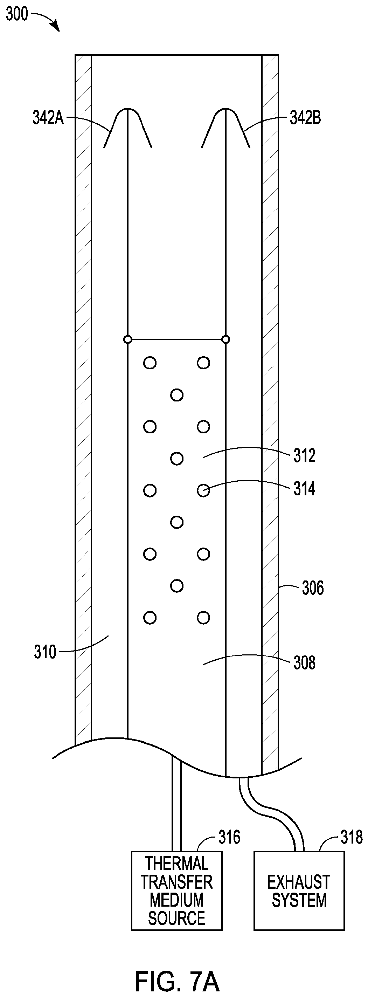

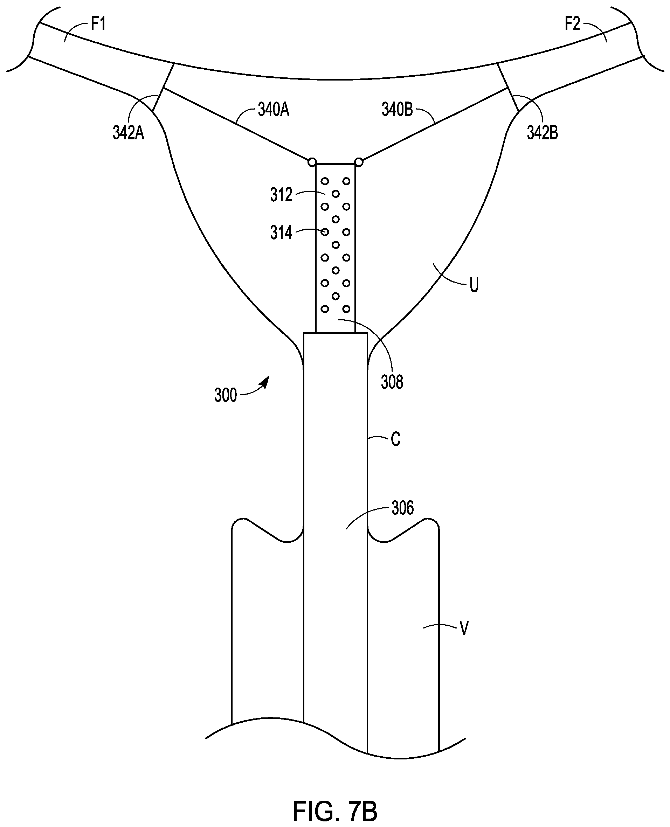

FIG. 7A is a schematic view of instrument 300, thermal transfer medium source 316, and exhaust system 318. FIG. 7B is a schematic view of a distal end of instrument 300 positioned in uterus U. Instrument 300 includes outer shaft 306, inner shaft 308, gap 310, nozzle 312, and one or more holes 314. FIG. 7A also shows thermal transfer medium source 316 and exhaust system 318. FIGS. 7A-7B further show first arm 340A, second arm 340B, first protector 342A, and second protector 342B of instrument 300. FIG. 7B also shows vagina V, cervix C, uterus U, first fallopian tube F1, and second fallopian tube F2.

Instrument 300 has generally the same structure and design as instrument 100 described above in reference to FIGS. 1A-1C, however instrument 300 also includes first arm 340A, second arm 340B, first protector 342A, and second protector 342B. First arm 340A, second arm 340B, first protector 342A, and second protector 342B are described here with respect to instrument 300, but can also be included on any of the embodiments of an instrument descried herewith, including instruments 100, 200, 400, 500, 600, 700, 800, 900, 1000, 1100, 1200, 1300, 1400, 1500, and 1600.

Instrument 300 can include a hand piece and a trigger, not shown in FIGS. 7A-7B. Outer shaft 306 of instrument 300 is configured to be inserted through vagina V and cervix C and into uterus U. Inner shaft 308 is configured to be deployed from outer shaft 306 when a distal end of outer shaft 306 is positioned in uterus U. Gap 310 is formed between an outer surface of inner shaft 308 and an inner surface of outer shaft 306. Nozzle 312 forms a distal end of inner shaft 308 and includes one or more holes 314 that are configured to deliver a thermal transfer medium to uterus U. Any suitable thermal transfer medium, such as those discussed above in reference to FIGS. 2-5, can be delivered to uterus U.

Thermal transfer medium source 316 is fluidly coupled to inner shaft 308 and is configured to deliver a thermal transfer medium to uterus U through inner shaft 308, nozzle 312, and one or more holes 314. Exhaust system 318 is fluidly coupled to outer shaft 306 and is configured to exhaust the thermal transfer medium from uterus U through outer shaft 306.

Instrument 300 further includes first arm 340A, second arm 340B, first protector 342A, and second protector 342B. In alternate embodiments, instrument 300 can include a single arm and/or a single protector. First arm 340A and second arm 340B are connected to a distal end of nozzle 312. First arm 340A has first protector 342A at a distal end. Second arm 340B has second protector 342B at a distal end. As shown in FIG. 7A, when instrument 300 is in a stowed position, first arm 340A, second arm 340B, first protector 342A, and second protector 342B are held in outer shaft 306. When a distal end of outer shaft 306 is positioned in uterus U and instrument 300 is deployed, nozzle 312, first arm 340A, second arm 340B, first protector 342A, and second protector 342B are moved out of outer shaft 306. As first arm 340A, second arm 340B, first protector 342A, and second protector 342B are moved out of outer shaft 306, first arm 340A and second arm 340B will move outwards. First arm 340A will move towards first fallopian tube F1, and second arm 340B will move towards second fallopian tube F2. First protector 342A at the distal end of first arm 340A will cover the opening to first fallopian tube F1, and second protector 342B at the distal end of second arm 340B will cover the opening to second fallopian tube F2. First protector 342A and second protector 342B block first fallopian tube F1 and second fallopian tube F2, respectively, to prevent the thermal transfer medium in uterus U from flowing through first fallopian tube F1 and second fallopian tube F2.

First arm 340A and second arm 340B can move outwards towards first fallopian tube F1 and second fallopian tube F2 using any suitable mechanism. For example, first arm 340A and second arm 340B can spring outwards. By further example, first arm 340A and second arm 340B can be guided outwards with guide wires. Additionally, first arm 340A, second arm 340B, first protector 342A, and second protector 342B can include echogenic materials so that ultrasound can be used to guide the placement of first arm 340A, second arm 340B, first protector 342A, and second protector 342B.

First protector 342A and second protector 342B are shown as being covers in the embodiment shown in FIGS. 7A-7B. First protector 342A and second protector 342B can include any suitable mechanism that is capable of blocking first fallopian tube F1 and second fallopian tube F2. Additional examples of protectors 342 will be discussed in reference to instruments 400, 500, 600, 700, and 800.

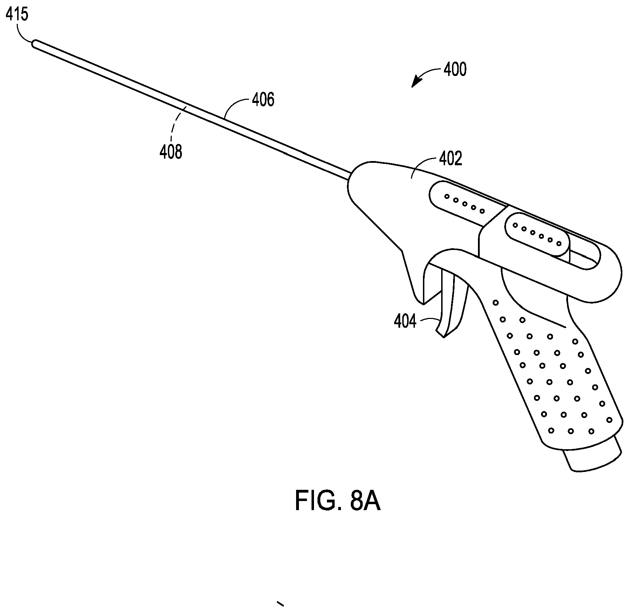

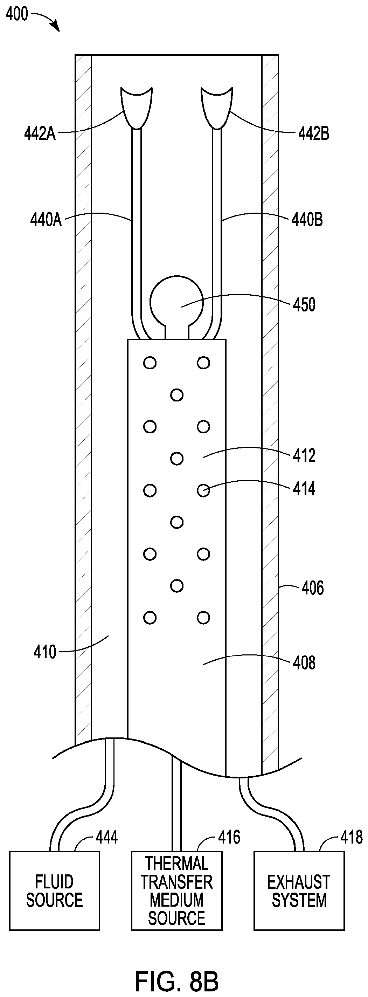

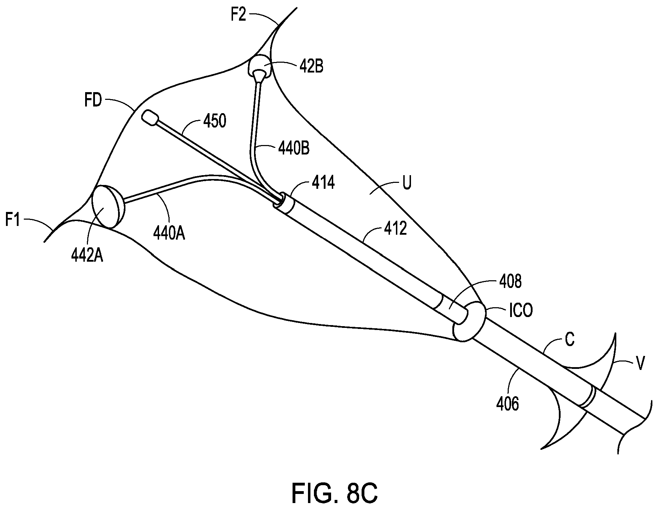

FIG. 8A is a perspective view of instrument 400. FIG. 8B is a schematic view of instrument 400, fluid source 432, thermal transfer medium source 416, and exhaust system 418. FIG. 8C is a schematic view of a distal end of instrument 400 positioned in uterus U. Instrument 400 includes hand piece 402, trigger 404, depth indicator 405, outer shaft 406, inner shaft 408, gap 410, nozzle 412, one or more holes 414, and gauge 415. FIG. 8A also shows thermal transfer medium source 416 and exhaust system 418. FIGS. 8A-8B further show first arm 440A, second arm 440B, first inflatable member 442A, and second inflatable member 442B of instrument 400, and FIG. 8B shows fluid source 444. FIG. 8B also shows vagina V, cervix C, internal cervical os ICO, uterus U, first fallopian tube F1, second fallopian tube F2, and fundus FD.

Instrument 400 has generally the same structure and design as instrument 100 described above in reference to FIGS. 1A-1C, however instrument 400 also includes depth indicator 405, gauge 415, first arm 440A, second arm 440B, first inflatable member 442A, second inflatable member 442B, and fluid source 444. Depth indicator 405, gauge 415, first arm 440A, second arm 440B, first inflatable member 442A, second inflatable member 442B, and fluid source 444 are described here with respect to instrument 400, but can also be included on any of the embodiments of an instrument descried herewith, including instruments 100, 200, 300, 500, 600, 700, 800, 900, 1000, 1100, 1200, 1300, 1400, 1500, and 1600.

Hand piece 402 forms a body portion of instrument 400 that is designed to be grasped by a user. Trigger 404 is connected to hand piece 402 and is positioned to be pulled by a user when the user is grasping hand piece 402. Hand piece 402 further includes depth indicator 405. Outer shaft 406 of instrument 400 is configured to be inserted through vagina V and cervix C and into uterus U. Inner shaft 408 is configured to be deployed from outer shaft 406 when a distal end of outer shaft 406 is positioned in uterus U. Gap 410 is formed between an outer surface of inner shaft 408 and an inner surface of outer shaft 406. Nozzle 412 forms a distal end of inner shaft 408 and includes one or more holes 414 that are configured to deliver a thermal transfer medium to uterus U. Any suitable thermal transfer medium, such as those discussed above in reference to FIGS. 2-5, can be delivered to uterus U.

Instrument 400 also includes gauge 415. Gauge 415 extends from a distal end of nozzle 412. Gauge 415 can be advanced forward out of nozzle 412 when instrument 400 is positioned in uterus U until gauge 415 comes into contract with fundus FD of uterus U, as shown in FIG. 8C. Gauge 415 and depth indicator 405 can be used to determine the distance through uterus U from internal cervical os ICO to fundus FD. Gauge 415 and depth indicator 405 can include any suitable mechanism that is capable of determining the distance through uterus U.

Thermal transfer medium source 416 is fluidly coupled to inner shaft 408 and is configured to deliver a thermal transfer medium to uterus U through inner shaft 408, nozzle 412, and one or more holes 414. Exhaust system 418 is fluidly coupled to outer shaft 406 and is configured to exhaust the thermal transfer medium from uterus U through outer shaft 406.

Instrument 400 further includes first arm 440A, second arm 440B, first inflatable member 442A, and second inflatable member 442B. In alternate embodiments, instrument 400 can include a single arm and/or a single inflatable member. First arm 440A and second arm 440B are connected to a distal end of nozzle 412. First arm 440A has first inflatable member 442A at a distal end. Second arm 440B has second inflatable member 442B at a distal end. As shown in FIG. 8B, when instrument 400 is in a stowed position, first arm 440A, second arm 440B, first inflatable member 442A, and second inflatable member 442B are held in outer shaft 406. When a distal end of outer shaft 406 is positioned in uterus U and instrument 400 is deployed, nozzle 412, first arm 440A, second arm 440B, first inflatable member 442A, and second inflatable member 442B are moved out of outer shaft 406. As first arm 440A, second arm 440B, first inflatable member 442A, and second inflatable member 442B are moved out of outer shaft 406, first arm 440A and second arm 440B will move outwards. First arm 440A will move towards first fallopian tube F1, and second arm 440B will move towards second fallopian tube F2. First inflatable member 442A at the distal end of first arm 440A will cover the opening to first fallopian tube F1, and second inflatable member 442B at the distal end of second arm 440B will cover the opening to second fallopian tube F2. First inflatable member 442A and second inflatable member 442B are connected to fluid source 444, as shown in FIG. 8B. Fluid source 444 is configured to provide a flow of fluid to first inflatable member 442A and second inflatable member 442B to cause first inflatable member 442A and second inflatable member 442B to expand to block first fallopian tube F1 and second fallopian tube F2, respectively, to prevent the thermal transfer medium in uterus U from flowing through first fallopian tube F1 and second fallopian tube F2.

First arm 440A and second arm 440B can move outwards towards first fallopian tube F1 and second fallopian tube F2 using any suitable mechanism. For example, first arm 440A and second arm 440B can spring outwards. By further example, first arm 440A and second arm 440B can be guided outwards with guide wires. Additionally, first arm 440A, second arm 440B, first inflatable member 442A, and second inflatable member 442B can include echogenic materials so that ultrasound can be used to guide the placement of first arm 440A, second arm 440B, first inflatable member 442A, and second inflatable member 442B.

First inflatable member 442A and second inflatable member 442B are one embodiment of a protector to protect first fallopian tube F1 and second fallopian tube F2. Additional embodiments of protectors are discussed in reference to instruments 300, 500, 600, 700, and 800.

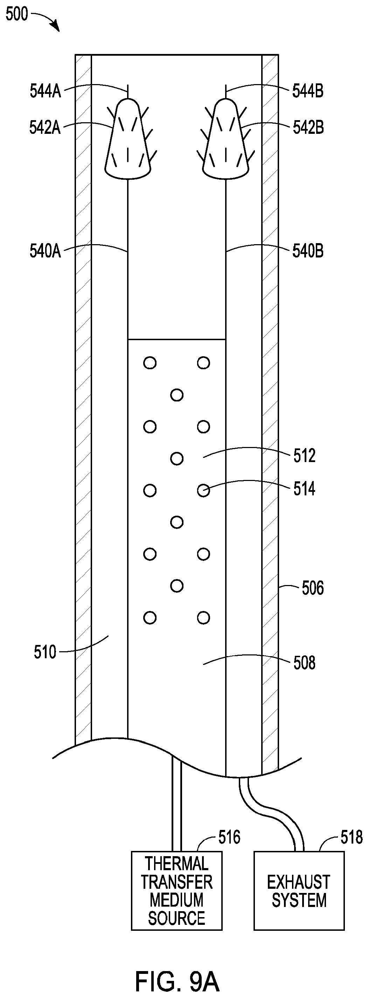

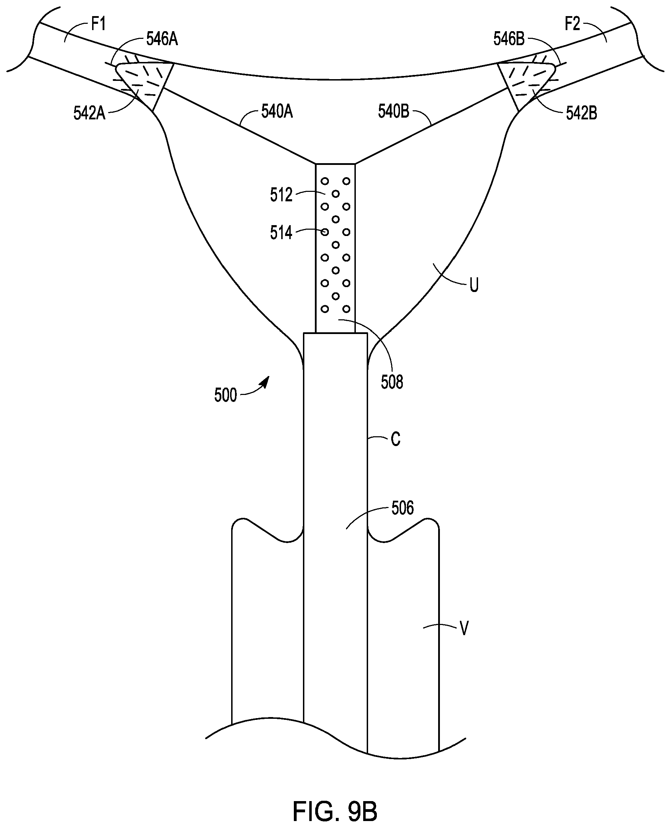

FIG. 9A is a schematic view of instrument 500, thermal transfer medium source 516, and exhaust system 518. FIG. 9B is a schematic view of a distal end of instrument 500 positioned in uterus U. Instrument 500 includes outer shaft 506, inner shaft 508, gap 510, nozzle 512, and one or more holes 514. FIG. 9A also shows thermal transfer medium source 516 and exhaust system 518. FIGS. 9A-9B further show first arm 540A, second arm 540B, first friction enhancing member 542A, second friction enhancing member 542B, first retention members 546A, and second retention members 546B of instrument 500. FIG. 9B also shows vagina V, cervix C, uterus U, and first fallopian tube F1 and second fallopian tube F2.

Instrument 500 has generally the same structure and design as instrument 100 described above in reference to FIGS. 1A-1C, however instrument 500 also includes first arm 540A, second arm 540B, first friction enhancing member 542A, second friction enhancing member 542B, first retention members 546A, and second retention members 546B. First arm 540A, second arm 540B, first friction enhancing member 542A, second friction enhancing member 542B, first retention members 546A, and second retention members 546B are described here with respect to instrument 500, but can also be included on any of the embodiments of an instrument descried herewith, including instruments 100, 200, 300, 400, 600, 700, 800, 900, 1000, 1100, 1200, 1300, 1400, 1500, and 1600.

Instrument 500 can include a hand piece and a trigger, not shown in FIGS. 9A-9B. Outer shaft 506 of instrument 500 is configured to be inserted through vagina V and cervix C and into uterus U. Inner shaft 508 is configured to be deployed from outer shaft 506 when a distal end of outer shaft 506 is positioned in uterus U. Gap 510 is formed between an outer surface of inner shaft 508 and an inner surface of outer shaft 506. Nozzle 512 forms a distal end of inner shaft 508 and includes one or more holes 514 that are configured to deliver a thermal transfer medium to uterus U. Any suitable thermal transfer medium, such as those discussed above in reference to FIGS. 2-5, can be delivered to uterus U.

Thermal transfer medium source 516 is fluidly coupled to inner shaft 508 and is configured to deliver a thermal transfer medium to uterus U through inner shaft 508, nozzle 512, and one or more holes 514. Exhaust system 518 is fluidly coupled to outer shaft 506 and is configured to exhaust the thermal transfer medium from uterus U through outer shaft 506.

Instrument 500 further includes first arm 540A, second arm 540B, first friction enhancing member 542A, second friction enhancing member 542B, first retention members 546A, and second retention members 546B. In alternate embodiments, instrument 500 can include a single arm and/or a single friction enhancing member. First arm 540A and second arm 540B are connected to a distal end of nozzle 512. First arm 540A has first friction enhancing member 542A at a distal end. Second arm 540B has second friction enhancing member 542B at a distal end. First friction enhancing member 542A and second friction enhancing members 542B are shown as being plugs in the embodiment shown in FIGS. 9A-9B, but can be any suitable friction enhancing members in alternate embodiments. First retention members 546A and second retention members 546B extend from first friction enhancing member 542A and second friction enhancing member 542B, respectively. First retention members 546A and second retention members 546B are shown as being barbs in the embodiment shown in FIGS. 9A-9B, but can be any suitable retention member in alternate embodiments. As shown in FIG. 9A, when instrument 500 is in a stowed position, first arm 540A, second arm 540B, first friction enhancing member 542A, and second friction enhancing member 542B are held in outer shaft 506. When a distal end of outer shaft 506 is positioned in uterus U and instrument 500 is deployed, nozzle 512, first arm 540A, second arm 540B, first friction enhancing member 542A, and second friction enhancing member 542B are moved out of outer shaft 506. As first arm 540A, second arm 540B, first friction enhancing member 542A, and second friction enhancing member 542B are moved out of outer shaft 506, first arm 540A and second arm 54B will move outwards. First arm 540A will move towards first fallopian tube F1, and second arm 540B will move towards second fallopian tube F2. First friction enhancing member 542A at the distal end of first arm 540A can be inserted into the opening to first fallopian tube F1, and second friction enhancing member 542B at the distal end of second arm 540B can be inserted into the opening to second fallopian tube F2. First friction enhancing member 542A and second friction enhancing member 542B block first fallopian tube F1 and second fallopian tube F2, respectively, to prevent the thermal transfer medium in uterus U from flowing through first fallopian tube F1 and second fallopian tube F2. First retention members 546A and second retention members 546B on first friction enhancing member 542A and second friction enhancing member 542B, respectively, will engage the walls of first fallopian tube F1 and second fallopian tube F2 to help retain first friction enhancing member 542A and second friction enhancing member 542B in first fallopian tube F1 and second fallopian tube F2.

First arm 540A and second arm 540B can move outwards towards first fallopian tube F1 and second fallopian tube F2 using any suitable mechanism. For example, first arm 540A and second arm 540B can spring outwards. By further example, first arm 540A and second arm 540B can be guided outwards with guide wires. Additionally, first arm 540A, second arm 540B, first friction enhancing member 542A, and second friction enhancing member 542B can include echogenic materials so that ultrasound can be used to guide the placement of first arm 540A, second arm 540B, first friction enhancing member 542A, and second friction enhancing member 542B.

When instrument 500 is removed from uterus U, first friction enhancing member 542A and second friction enhancing member 542B can be disconnected from first arm 540A and second arm 540B, respectively. First retention members 546A and second retention members 546B will help retain first friction enhancing member 542A and second friction enhancing member 542B in first fallopian tube F1 and second fallopian tube F2, respectively, when first friction enhancing member 542A and second friction enhancing member 542B are disconnected from first arm 540A and second arm 540B. First friction enhancing member 542A and second friction enhancing member 542B can act as permanent birth control when they are retained in first fallopian tube F1 and second fallopian tube F2. As an egg is released from an ovary and travels along first fallopian tube F1 or second fallopian tube F2 towards uterus U, first friction enhancing member 542A and second friction enhancing member 542B will prevent the egg from entering uterus U.

First friction enhancing member 542A and second friction enhancing member 542B are one embodiment of a protector to protect first fallopian tube F1 and second fallopian tube F2. Additional embodiments of protectors are discussed in reference to instruments 300, 400, 600, 700, and 800.

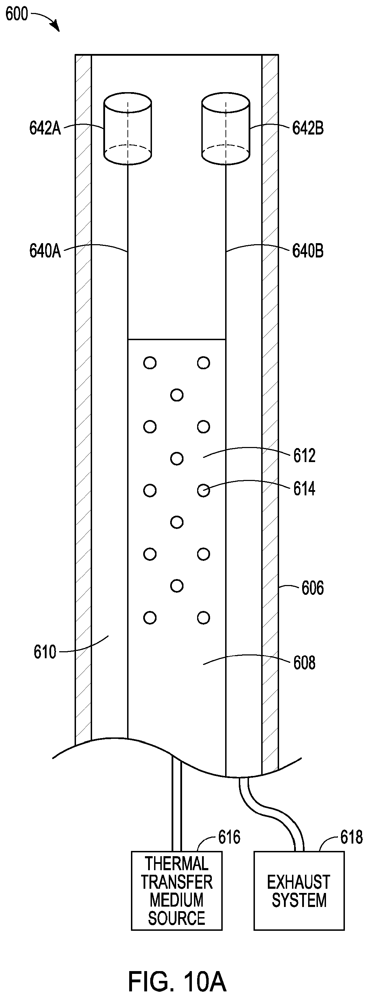

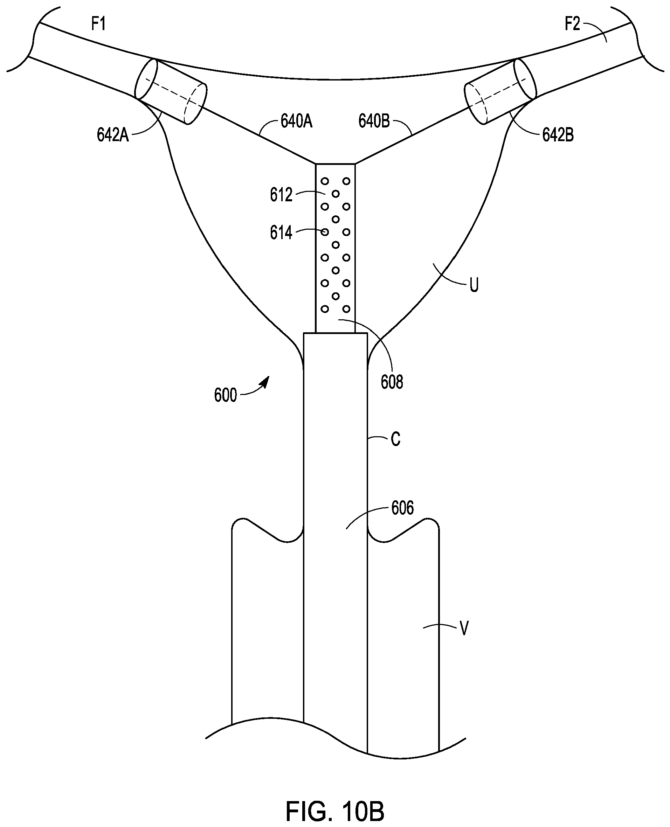

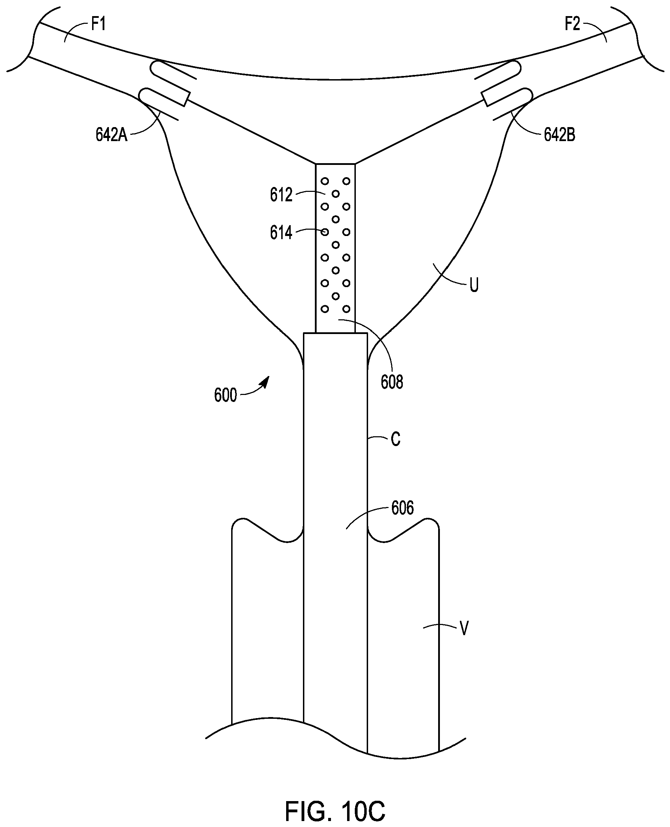

FIG. 10A is a schematic view of instrument 600, thermal transfer medium source 616, and exhaust system 618. FIG. 10B is a schematic view of a distal end of instrument 600 positioned in uterus U with unfurl members 642 in a furled state. FIG. 10C is a schematic view of a distal end of instrument 600 positioned in uterus U with unfurl members 642 in an unfurled state. Instrument 600 includes outer shaft 606, inner shaft 608, gap 610, nozzle 612, and one or more holes 614. FIG. 10A also shows thermal transfer medium source 616 and exhaust system 618. FIGS. 10A-10C further show first arm 640A, second arm 640B, first unfurl member 642A, and second unfurl member 642B of instrument 600. FIGS. 10B-10C also shows vagina V, cervix C, uterus U, first fallopian tube F1, and second fallopian tube F2.

Instrument 600 has generally the same structure and design as instrument 100 described above in reference to FIGS. 1A-1C, however instrument 600 also includes first arm 640A, second arm 640B, first unfurl member 642A, and second unfurl member 642B. First arm 640A, second arm 640B, first unfurl member 642A, and second unfurl member 642B described here with respect to instrument 600, but can also be included on any of the embodiments of an instrument descried herewith, including instruments 100, 200, 300, 400, 500, 700, 800, 900, 1000, 1100, 1200, 1300, 1400, 1500, and 1600.

Instrument 600 can include a hand piece and a trigger, not shown in FIGS. 10A-10C. Outer shaft 606 of instrument 600 is configured to be inserted through vagina V and cervix C and into uterus U. Inner shaft 608 is configured to be deployed from outer shaft 606 when a distal end of outer shaft 606 is positioned in uterus U. Gap 610 is formed between an outer surface of inner shaft 608 and an inner surface of outer shaft 606. Nozzle 612 forms a distal end of inner shaft 608 and includes one or more holes 614 that are configured to deliver a thermal transfer medium to uterus U. Any suitable thermal transfer medium, such as those discussed above in reference to FIGS. 2-5, can be delivered to uterus U.

Thermal transfer medium source 616 is fluidly coupled to inner shaft 608 and is configured to deliver a thermal transfer medium to uterus U through inner shaft 608, nozzle 612, and one or more holes 614. Exhaust system 618 is fluidly coupled to outer shaft 606 and is configured to exhaust the thermal transfer medium from uterus U through outer shaft 606.

Instrument 600 further includes first arm 640A, second arm 640B, first unfurl member 642A, and second unfurl member 642B. In alternate embodiments, instrument 600 can include a single arm and/or a single unfurl member. First arm 640A and second arm 640B are connected to a distal end of nozzle 612. First arm 640A has first unfurl member 642A at a distal end. Second arm 640B has second unfurl member 642B at a distal end. As shown in FIG. 10A, when instrument 600 is in a stowed position, first arm 640A, second arm 640B, first unfurl member 642A, and second unfurl member 642B are held in outer shaft 606. When a distal end of outer shaft 606 is positioned in uterus U and instrument 600 is deployed, nozzle 612, first arm 640A, second arm 640B, first unfurl member 642A, and second unfurl member 642B are moved out of outer shaft 606. As first arm 640A, second arm 640B, first unfurl member 642A, and second unfurl member 642B are moved out of outer shaft 606, first arm 640A and second arm 640B will move outwards. First arm 640A will move towards first fallopian tube F1, and second arm 640B will move towards second fallopian tube F2. First unfurl member 642A at the distal end of first arm 640A will be positioned at the opening to first fallopian tube F1 in a furled state, and second unfurl members 642B at the distal end of second arm 640B will be positioned at the opening to second fallopian tube F2 in a furled stated, as shown in FIG. 10B. First unfurl member 642A and second unfurl member 642B can then be unfurled by pulling first arm 640A and second arm 640B. First unfurl member 642A and second unfurl member 642B will block first fallopian tube F1 and second fallopian tube F2, respectively, in the unfurled state, as shown in FIG. 10C, to prevent the thermal transfer medium in uterus U from flowing through first fallopian tube F1 and second fallopian tube F2.

First arm 640A and second arm 640B can move outwards towards first fallopian tube F1 and second fallopian tube F2 using any suitable mechanism. For example, first arm 640A and second arm 640B can spring outwards. By further example, first arm 640A and second arm 640B can be guided outwards with guide wires. Additionally, first arm 640A, second arm 640B, first unfurl member 642A, and second unfurl member 642B can include echogenic materials so that ultrasound can be used to guide the placement of first arm 640A, second arm 640B, first unfurl member 642A, and second unfurl member 642B.

First unfurl member 642A and second unfurl member 642B are one embodiment of a protector to protect first fallopian tube F1 and second fallopian tube F2. Additional embodiments of protectors are discussed in reference to instruments 300, 400, 500, 700, and 800.

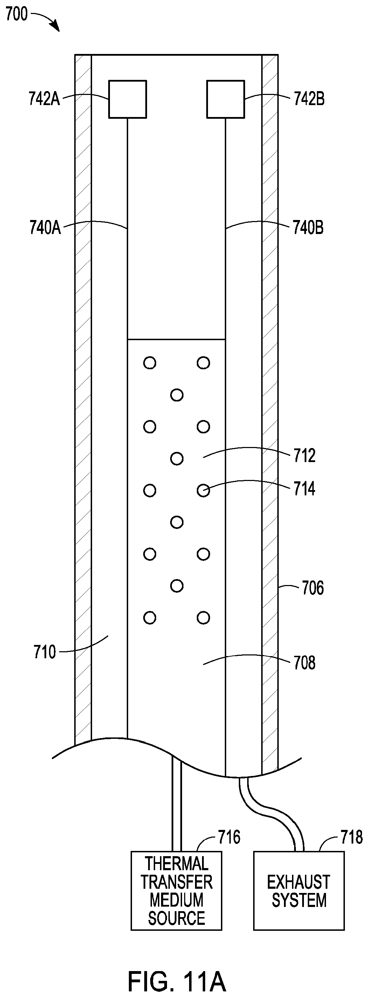

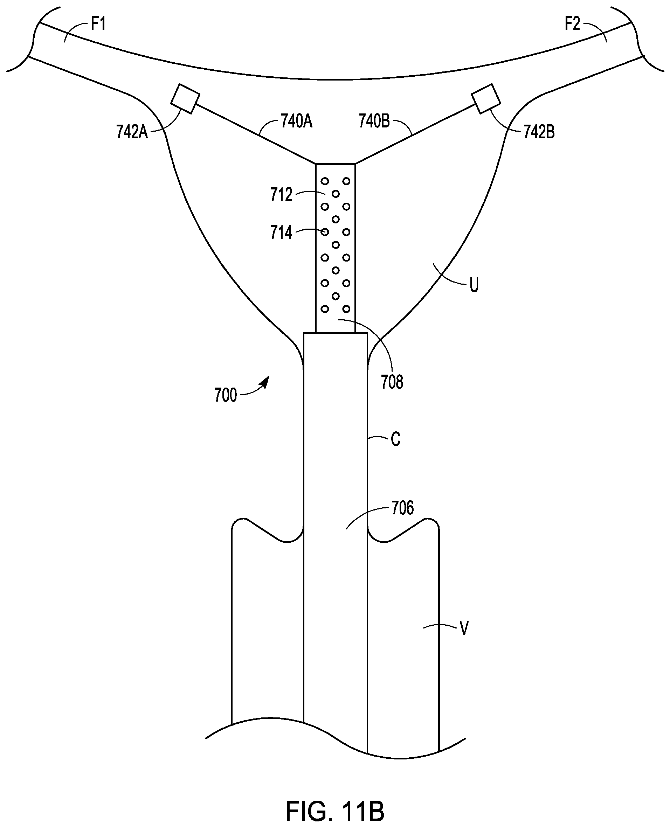

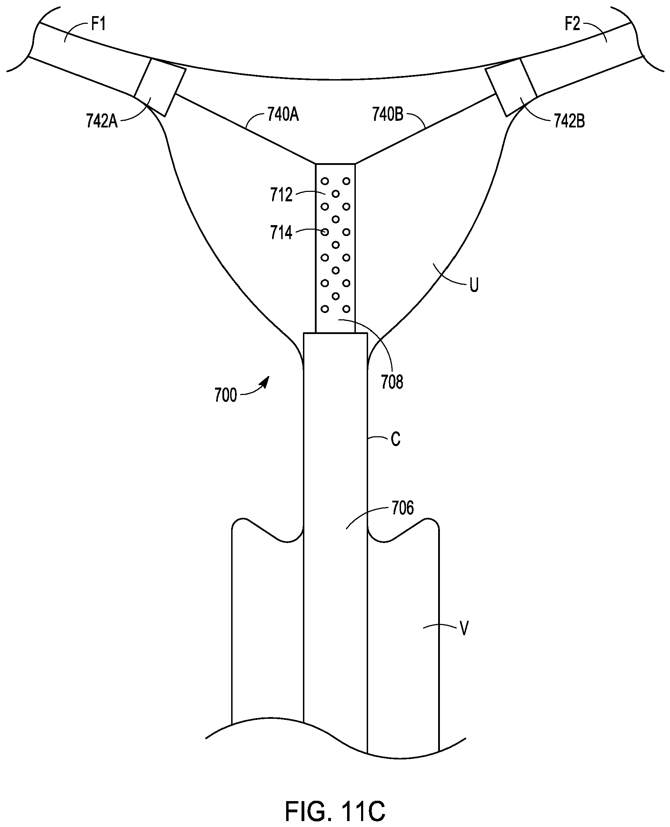

FIG. 11A is a schematic view of instrument 700, thermal transfer medium source 716, and exhaust system 718. FIG. 11B is a schematic view of a distal end of instrument 700 positioned in uterus U with pledgets 742 in an unexpanded state. FIG. 11C is a schematic view of a distal end of instrument 700 positioned in uterus U with pledgets 742 in an expanded state. Instrument 700 includes outer shaft 706, inner shaft 708, gap 710, nozzle 712, and one or more holes 714. FIG. 11A also shows thermal transfer medium source 716 and exhaust system 718. FIGS. 11A-11C further show first arm 740A, second arm 740B, first pledget 742A, and second pledget 742B of instrument 700. FIGS. 11B-11C also shows vagina V, cervix C, uterus U, and first fallopian tube F1 and second fallopian tube F2.

Instrument 700 has generally the same structure and design as instrument 100 described above in reference to FIGS. 1A-1C, however instrument 700 also includes first arm 740A, second arm 740B, first pledget 742A, and second pledget 742B. First arm 740A, second arm 740B, first pledget 742A, and second pledget 742B are described here with respect to instrument 700, but can also be included on any of the embodiments of an instrument descried herewith, including instruments 100, 200, 300, 400, 500, 600, 800, 900, 1000, 1100, 1200, 1300, 1400, 1500, and 1600.

Instrument 700 can include a hand piece and a trigger, not shown in FIGS. 11A-11C. Outer shaft 706 of instrument 700 is configured to be inserted through vagina V and cervix C and into uterus U. Inner shaft 708 is configured to be deployed from outer shaft 706 when a distal end of outer shaft 706 is positioned in uterus U. Gap 710 is formed between an outer surface of inner shaft 708 and an inner surface of outer shaft 706. Nozzle 712 forms a distal end of inner shaft 708 and includes one or more holes 714 that are configured to deliver a thermal transfer medium to uterus U. Any suitable thermal transfer medium, such as those discussed above in reference to FIGS. 2-5, can be delivered to uterus U.

Thermal transfer medium source 716 is fluidly coupled to inner shaft 708 and is configured to deliver a thermal transfer medium to uterus U through inner shaft 708, nozzle 712, and one or more holes 714. Exhaust system 718 is fluidly coupled to outer shaft 706 and is configured to exhaust the thermal transfer medium from uterus U through outer shaft 706.

Instrument 700 further includes first arm 740A, second arm 740B, first pledget 742A, and second pledget 742B. In alternate embodiments, instrument 700 can include a single arm and/or a single pledget. First arm 740A and second arm 740B are connected to a distal end of nozzle 712. First arm 740A has first pledget 742A at a distal end. Second arm 740B has second pledget 742B at a distal end. As shown in FIG. 11A, when instrument 700 is in a stowed position, first arm 740A, second arm 740B, first pledget 742A, and second pledget 742B are held in outer shaft 706. First pledget 742A and second pledget 742B are in an unexpanded state when they are held in outer shaft 706. When a distal end of outer shaft 706 is positioned in uterus U and instrument 700 is deployed, nozzle 712, first arm 740A, second arm 740B, first pledget 742A, and second pledget 742B are moved out of outer shaft 706. As first arm 740A, second arm 740B, first pledget 742A, and second pledget 742B are moved out of outer shaft 706, first arm 740A and second arm 740B will move outwards. First arm 740A will move towards first fallopian tube F1, and second arm 740B will move towards second fallopian tube F2. First pledget 742A at the distal end of first arm 740A will be positioned at the opening to first fallopian tube F1 in an unexpanded state, and second pledget 742B at the distal end of second arm 740B will be positioned at the opening to second fallopian tube F2 in unexpanded state, as shown in FIG. 11B. First pledget 742A and second pledget 742B can then absorb fluid from first fallopian tube F1, second fallopian tube F2, and uterus U to transition from the unexpanded state to an expanded state. First pledget 742A and second pledget 742B in an expanded state will block first fallopian tube F1 and second fallopian tube F2, respectively, as shown in FIG. 11C, to prevent the thermal transfer medium in uterus U from flowing through first fallopian tube F1 and second fallopian tube F2.

First arm 740A and second arm 740B can move outwards towards first fallopian tube F1 and second fallopian tube F2 using any suitable mechanism. For example, First arm 740A and second arm 740B can spring outwards. By further example, First arm 740A and second arm 740B can be guided outwards with guide wires. Additionally, first arm 740A, second arm 740B, first pledget 742A, and second pledget 742B can include echogenic materials so that ultrasound can be used to guide the placement of first arm 740A, second arm 740B, first pledget 742A, and second pledget 742B.

First pledget 742A and second pledget 742B are one embodiment of a protector to protect first fallopian tube F1 and second fallopian tube F2. Additional embodiments of protectors are discussed in reference to instruments 300, 400, 500, 600, and 800.

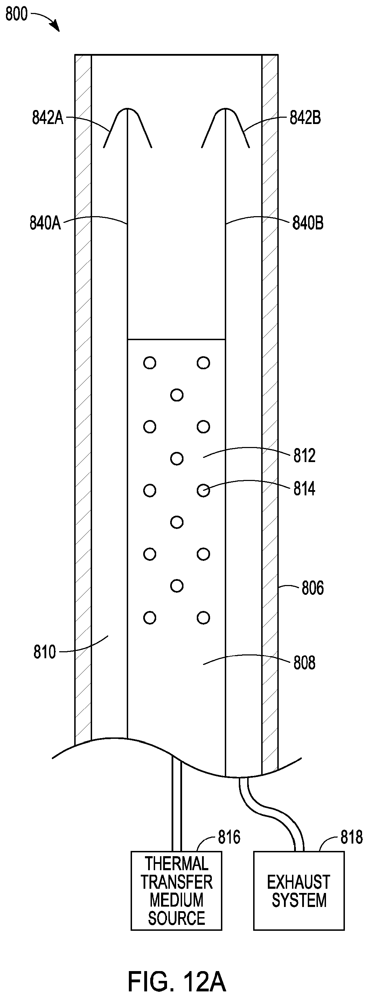

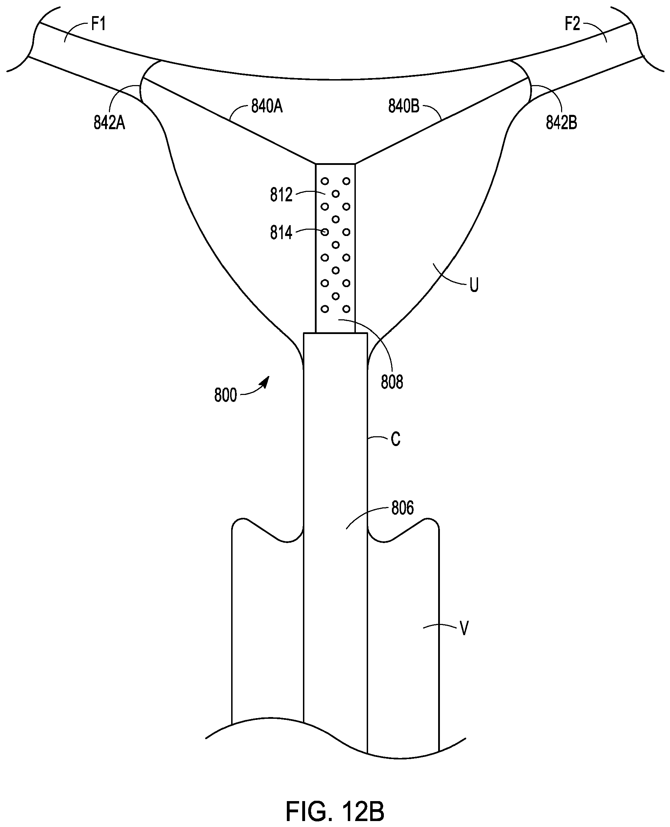

FIG. 12A is a schematic view of instrument 800, thermal transfer medium source 816, and exhaust system 818. FIG. 12B is a schematic view of a distal end of instrument 800 positioned in uterus U. Instrument 800 includes outer shaft 806, inner shaft 808, gap 810, nozzle 812, and one or more holes 814. FIG. 12A also shows thermal transfer medium source 816 and exhaust system 818. FIGS. 12A-12B further show first arm 840A, second arm 840B, first self-seeking and self-limiting member 842A, and second self-seeking and self-limiting member 842B of instrument 800. FIG. 12B also shows vagina V, cervix C, uterus U, and first fallopian tube F1 and second fallopian tube F2.

Instrument 800 has generally the same structure and design as instrument 100 described above in reference to FIGS. 1A-1C, however instrument 800 also includes first arm 840A, second arm 840B, first self-seeking and self-limiting member 842A, and second self-seeking and self-limiting member 842B. First arm 840A, second arm 840B, first self-seeking and self-limiting member 842A, and second self-seeking and self-limiting member 842B are described here with respect to instrument 800, but can also be included on any of the embodiments of an instrument descried herewith, including instruments 100, 200, 300, 400, 500, 600, 700, 900, 1000, 1100, 1200, 1300, 1400, 1500, and 1600.

Instrument 800 can include a hand piece and a trigger, not shown in FIGS. 12A-12B. Outer shaft 806 of instrument 800 is configured to be inserted through vagina V and cervix C and into uterus U. Inner shaft 808 is configured to be deployed from outer shaft 806 when a distal end of outer shaft 806 is positioned in uterus U. Gap 810 is formed between an outer surface of inner shaft 808 and an inner surface of outer shaft 806. Nozzle 812 forms a distal end of inner shaft 808 and includes one or more holes 814 that are configured to deliver a thermal transfer medium to uterus U. Any suitable thermal transfer medium, such as those discussed above in reference to FIGS. 2-5, can be delivered to uterus U.

Thermal transfer medium source 816 is fluidly coupled to inner shaft 808 and is configured to deliver a thermal transfer medium to uterus U through inner shaft 808, nozzle 812, and one or more holes 814. Exhaust system 818 is fluidly coupled to outer shaft 806 and is configured to exhaust the thermal transfer medium from uterus U through outer shaft 806.

Instrument 800 further includes first arm 840A, second arm 840B, first self-seeking and self-limiting member 842A, and second self-seeking and self-limiting member 842B. In alternate embodiments, instrument 800 can include a single arm and/or a single self-seeking and self-limiting member. First arm 840A and second arm 840B are connected to a distal end of nozzle 812. First arm 840A has first self-seeking and self-limiting member 842A at a distal end. Second arm 840B has second self-seeking and self-limiting member 842B at a distal end. First self-seeking and self-limiting member 842A, and second self-seeking and self-limiting member 842B are pressurized flaps made from silicone or urethane in the embodiment shown in FIGS. 12A-12B, but can be any suitable self-seeking and self-limiting members in alternate embodiments. As shown in FIG. 12A, when instrument 800 is in a stowed position, first arm 840A, second arm 840B, first self-seeking and self-limiting member 842A, and second self-seeking and self-limiting member 842B are held in outer shaft 806. When a distal end of outer shaft 806 is positioned in uterus U and instrument 800 is deployed, nozzle 812, first arm 840A, second arm 840B, first self-seeking and self-limiting member 842A, and second self-seeking and self-limiting member 842B are moved out of outer shaft 806. As first arm 840A, second arm 840B, first self-seeking and self-limiting member 842A, and second self-seeking and self-limiting member 842B are moved out of outer shaft 806, first arm 840A and second arm 840B will move outwards. First arm 840A will move towards first fallopian tube F1, and second arm 840B will move towards second fallopian tube F2. First self-seeking and self-limiting member 842A at the distal end of first arm 840A will cover the opening to first fallopian tube F1, and second self-seeking and self-limiting member 842B at the distal end of second arm 840B will cover the opening to second fallopian tube F2. First self-seeking and self-limiting member 842A and second self-seeking and self-limiting member 842B can be pressurized flaps that will be extend into first fallopian tube F1 and second fallopian tube F2 when uterus U is filled with a thermal transfer medium. First self-seeking and self-limiting member 842A and second self-seeking and self-limiting member 842B block first fallopian tube F1 and second fallopian tube F2, respectively, to prevent the thermal transfer medium in uterus U from flowing down first fallopian tube F1 and second fallopian tube F2.

First arm 840A and second arm 840B can move outwards towards first fallopian tube F1 and second fallopian tube F2 using any suitable mechanism. For example, first arm 840A and second arm 840B can spring outwards. By further example, first arm 840A and second arm 840B can be guided outwards with guide wires. Additionally, first arm 840A, second arm 840B, first self-seeking and self-limiting member 842A, and second self-seeking and self-limiting member 842B can include echogenic materials so that ultrasound can be used to guide the placement of first arm 840A, second arm 840B, first self-seeking and self-limiting member 842A, and second self-seeking and self-limiting member 842B.

First self-seeking and self-limiting member 842A and second self-seeking and self-limiting member 842B are one embodiment of a protector to protect first fallopian tube F1 and second fallopian tube F2. Additional embodiments of protectors are discussed in reference to instruments 300, 400, 500, 600, and 700.

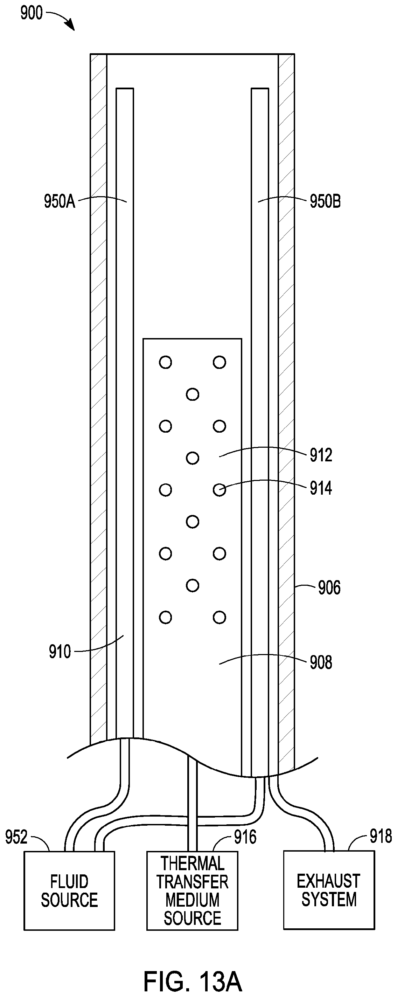

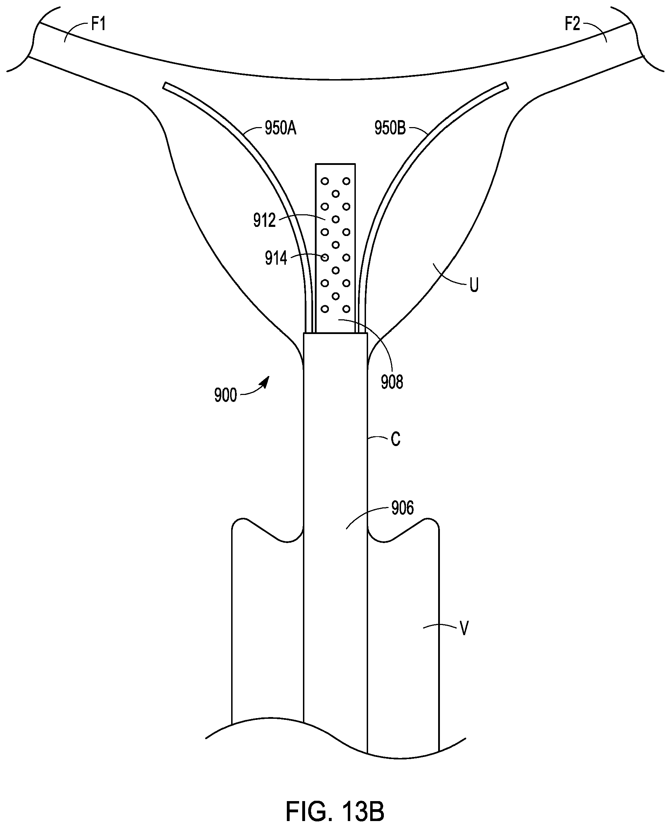

FIG. 13A is a schematic view of instrument 900, fluid source 352, thermal transfer medium source 916, and exhaust system 918. FIG. 13B is a schematic view of a distal end of instrument 900 positioned in uterus U. Instrument 900 includes outer shaft 906, inner shaft 908, gap 910, nozzle 912, and one or more holes 914. FIG. 13A also shows thermal transfer medium source 916 and exhaust system 918. FIGS. 13A-13B further show first tube 950A and second tube 950B of instrument 900, and FIG. 13A shows fluid source 952. FIG. 13B also shows vagina V, cervix C, uterus U, and first fallopian tube F1 and second fallopian tube F2.

Instrument 900 has generally the same structure and design as instrument 100 described above in reference to FIGS. 1A-1C, however instrument 900 also includes first tube 950A, second tube 950B, and fluid source 952. First tube 950A, second tube 950B, and fluid source 952 are described here with respect to instrument 900, but can also be included on any of the embodiments of an instrument descried herewith, including instruments 100, 200, 300, 400, 500, 600, 700, 800, 1000, 1100, 1200, 1300, 1400, 1500, and 1600.

Instrument 900 can include a hand piece and a trigger, not shown in FIGS. 13A-13B. Outer shaft 906 of instrument 900 is configured to be inserted through vagina V and cervix C and into uterus U. Inner shaft 908 is configured to be deployed from outer shaft 906 when a distal end of outer shaft 906 is positioned in uterus U. Gap 910 is formed between an outer surface of inner shaft 908 and an inner surface of outer shaft 906. Nozzle 912 forms a distal end of inner shaft 908 and includes one or more holes 914 that are configured to deliver a thermal transfer medium to uterus U. Any suitable thermal transfer medium, such as those discussed above in reference to FIGS. 2-5, can be delivered to uterus U.

Thermal transfer medium source 916 is fluidly coupled to inner shaft 908 and is configured to deliver a thermal transfer medium to uterus U through inner shaft 908, nozzle 912, and one or more holes 914. Exhaust system 918 is fluidly coupled to outer shaft 906 and is configured to exhaust the thermal transfer medium from uterus U through outer shaft 906.

Instrument 900 further includes first tube 950A and second tube 950B extending through outer shaft 906 in the embodiment shown in FIGS. 13A-13B. In alternate embodiments, instrument 900 can include a single tube. In alternate embodiments, first tube 950A and second tube 950B can extend through inner shaft 908 and nozzle 912. As shown in FIG. 13A, first tube 950A and second tube 950B are fluidly coupled to fluid source 952. As further shown in FIG. 13A, when instrument 900 is in a stowed position, nozzle 912, first tube 950A, and second tube 950B are positioned in outer shaft 906. When a distal end of outer shaft 906 is positioned in uterus U and instrument 900 is deployed, nozzle 912, first tube 950A, and second tube 950B are moved out of outer shaft 906. As first tube 950A and second tube 950B are moved out of outer shaft 906, first tube 950A and second tube 950B will move outwards. A distal end of first tube 950A will be positioned at an opening to first fallopian tube F1, and a distal end of second tube 950B will be positioned at an opening to second fallopian tube F2. As a thermal transfer medium flows into uterus U through one or more holes 914 in nozzle 912, a fluid from fluid source 952 can flow through first tube 950A and second tube 950B towards the openings of first fallopian tube F1 and second fallopian tube F2. The flow of fluid from first tube 950A and second tube 950B towards the openings of first fallopian tube F1 and second fallopian tube F2 deters the thermal transfer medium in uterus U from flowing down first fallopian tube F1 and second fallopian tube F2.

First tube 950A and second tube 950B can move outwards towards first fallopian tube F1 and second fallopian tube F2 using any suitable mechanism. For example, first tube 950A and second tube 950B can spring outwards. By further example, first tube 950A and second tube 950B can be guided outwards with guide wires. Additionally, first tube 950A and second tube 950B can include an echogenic material so that ultrasound can be used to guide the placement of first tube 950A and second tube 950B.

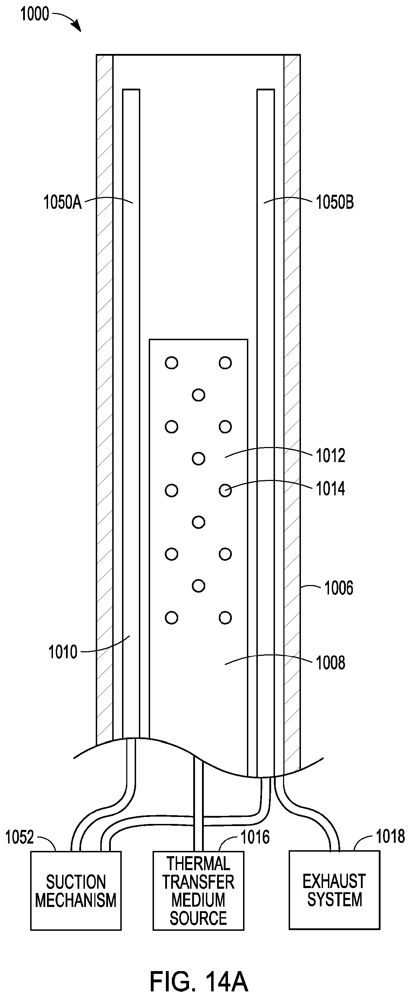

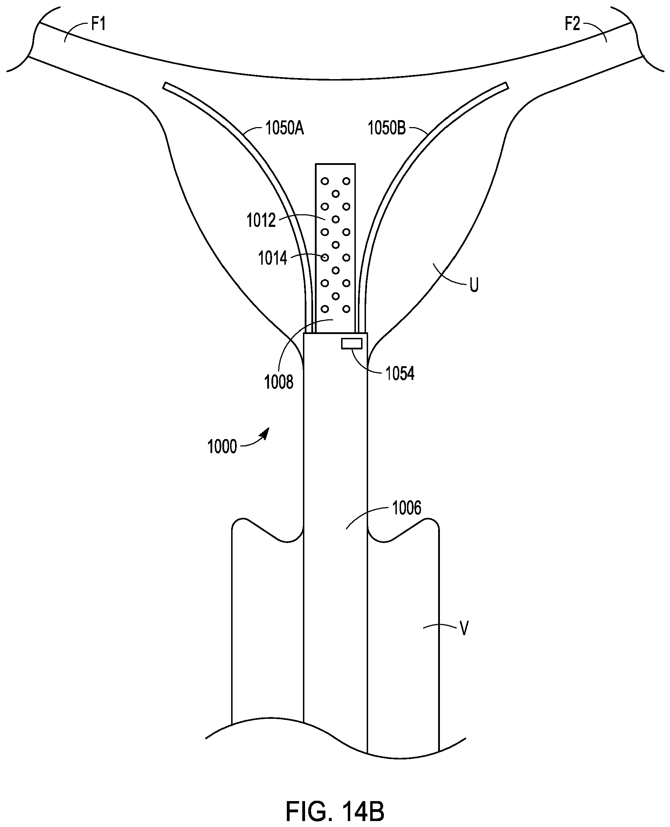

FIG. 14A is a schematic view of instrument 1000, fluid source 1052, thermal transfer medium source 1016, and exhaust system 1018. FIG. 14B is a schematic view of a distal end of instrument 1000 positioned in uterus U. Instrument 1000 includes outer shaft 1006, inner shaft 1008, gap 1010, nozzle 1012, and one or more holes 1014. FIG. 14A also shows thermal transfer medium source 1016 and exhaust system 1018. FIGS. 14A-14B further show first tube 1050A and second tube 1050B of instrument 1000, FIG. 14A shows suction mechanism 1052, and FIG. 14B shows pressure regulator 1054 of instrument 1000. FIG. 14B also shows vagina V, cervix C, uterus U, and first fallopian tube F1 and second fallopian tube F2.

Instrument 1000 has generally the same structure and design as instrument 100 described above in reference to FIGS. 1A-1C, however instrument 1000 also includes first tube 1050A, second tube 1050B, suction mechanism 1052, and pressure regulator 1054. First tube 1050A, second tube 1050B, suction mechanism 1052, and pressure regulator 1054 are described here with respect to instrument 1000, but can also be included on any of the embodiments of an instrument descried herewith, including instruments 100, 200, 300, 400, 500, 600, 700, 800, 900, 1100, 1200, 1300, 1400, 1500, and 1600.

Instrument 1000 can include a hand piece and a trigger, not shown in FIGS. 14A-14B. Outer shaft 1006 of instrument 1000 is configured to be inserted through vagina V and cervix C and into uterus U. Inner shaft 1008 is configured to be deployed from outer shaft 1006 when a distal end of outer shaft 1006 is positioned in uterus U. Gap 1010 is formed between an outer surface of inner shaft 1008 and an inner surface of outer shaft 1006. Nozzle 1012 forms a distal end of inner shaft 1008 and includes one or more holes 1014 that are configured to deliver a thermal transfer medium to uterus U. Any suitable thermal transfer medium, such as those discussed above in reference to FIGS. 2-5, can be delivered to uterus U.

Thermal transfer medium source 1016 is fluidly coupled to inner shaft 1008 and is configured to deliver a thermal transfer medium to uterus U through inner shaft 1008, nozzle 1012, and one or more holes 1014. Exhaust system 1018 is fluidly coupled to outer shaft 1006 and is configured to exhaust the thermal transfer medium from uterus U through outer shaft 1006.

Instrument 1000 further includes first tube 1050A and second tube 1050B extending through outer shaft 1006 in the embodiment shown in FIGS. 14A-14B. In alternate embodiments, instrument 1000 can include a single tube. In alternate embodiments, first tube 1050A and second tube 1050B can extend through inner shaft 1008 and nozzle 1012. As shown in FIG. 14A, first tube 1050A and second tube 1050B are fluidly coupled to suction mechanism 1052. As further shown in FIG. 14A, when instrument 1000 is in a stowed position, nozzle 1012, first tube 1050A, and second tube 1050B are positioned in outer shaft 1006. When a distal end of outer shaft 1006 is positioned in uterus U and instrument 1000 is deployed, nozzle 1012, first tube 1050A, and second tube 1050B are moved out of outer shaft 1006. As first tube 1050A and second tube 1050B are moved out of outer shaft 1006, first tube 1050A and second tube 1050B will move outwards. A distal end of first tube 1050A will be positioned at an opening to first fallopian tube F1, and a distal end of second tube 1050B will be positioned at an opening to second fallopian tube F2. As a thermal transfer medium flows into uterus U through one or more holes 1014 in nozzle 1012, suction mechanism 1052 can suck the thermal transfer medium at the openings of first fallopian tube F1 and second fallopian tube F2 into first tube 1050A and second tube 1050B to prevent it from flowing through first fallopian tube F1 and second fallopian tube F2.

Instrument 1000 also includes pressure regulator 1054. Pressure regulator 1054 can act as a control valve that can control the flow rate of thermal transfer medium into uterus U. Pressure regulator 1054 is shown as being positioned at the distal end of outer shaft 1006 in the embodiment shown in FIGS. 14A-14B, but can be positioned at other locations on outer shaft 1006 in alternate embodiments. If the pressure in uterus U gets too high, uterus U may perforate. Pressure regulator 1054 can control an inflow rate and an outflow rate of the thermal transfer medium to prevent uterine perforation. If the inflow rate and/or the outflow rate fall outside of a predetermined range, either an alarm can signal that the procedure needs to be stopped or pressure regulator 1054 can send a signal to a controller to stop the flow of the thermal transfer medium into uterus U.

First tube 1050A and second tube 1050B can move outwards towards first fallopian tube F1 and second fallopian tube F2 using any suitable mechanism. For example, first tube 1050A and second tube 1050B can spring outwards. By further example, first tube 1050A and second tube 1050B can be guided outwards with guide wires. Additionally, first tube 1050A and second tube 1050B can include an echogenic material so that ultrasound can be used to guide the placement of first tube 1050A and second tube 1050B.

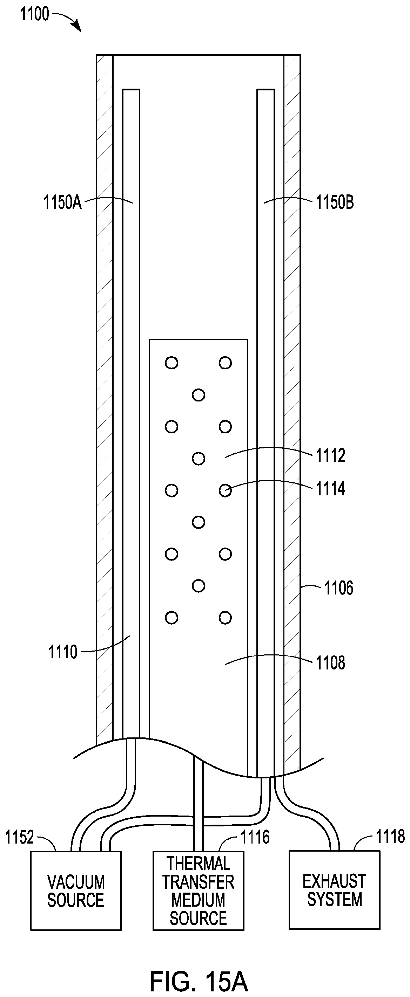

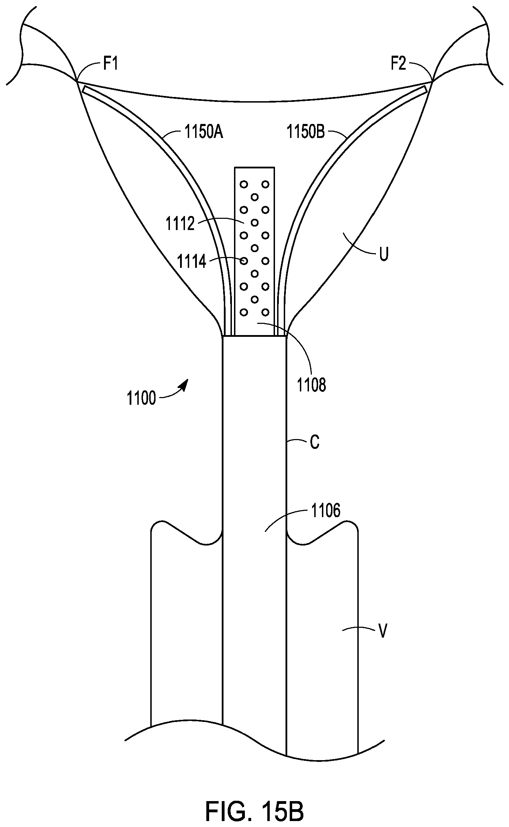

FIG. 15A is a schematic view of instrument 1100, vacuum source 1152, thermal transfer medium source 1116, and exhaust system 1118. FIG. 15B is a schematic view of a distal end of instrument 1100 positioned in uterus U. Instrument 1100 includes outer shaft 1106, inner shaft 1108, gap 1110, nozzle 1112, and one or more holes 1114. FIG. 15A also shows thermal transfer medium source 1116 and exhaust system 1118. FIGS. 15A-15B further show first tube 1150A and second tube 1150B of instrument 1100, and FIG. 15A shows vacuum source 1152. FIG. 15B also shows vagina V, cervix C, uterus U, and first fallopian tube F1 and second fallopian tube F2.

Instrument 1100 has generally the same structure and design as instrument 100 described above in reference to FIGS. 1A-1C, however instrument 1100 also includes first tube 1150A, second tube 1150B, and vacuum source 1152. First tube 1150A, second tube 1150B, and vacuum source 1152 are described here with respect to instrument 1100, but can also be included on any of the embodiments of an instrument descried herewith, including instruments 100, 200, 300, 400, 500, 600, 700, 800, 900, 1000, 1200, 1300, 1400, 1500, and 1600.

Instrument 1100 can include a hand piece and a trigger, not shown in FIGS. 15A-15B. Outer shaft 1106 of instrument 1100 is configured to be inserted through vagina V and cervix C and into uterus U. Inner shaft 1108 is configured to be deployed from outer shaft 1106 when a distal end of outer shaft 1106 is positioned in uterus U. Gap 1110 is formed between an outer surface of inner shaft 1108 and an inner surface of outer shaft 1106. Nozzle 1112 forms a distal end of inner shaft 1108 and includes one or more holes 1114 that are configured to deliver a thermal transfer medium to uterus U. Any suitable thermal transfer medium, such as those discussed above in reference to FIGS. 2-5, can be delivered to uterus U.

Thermal transfer medium source 1116 is fluidly coupled to inner shaft 1108 and is configured to deliver a thermal transfer medium to uterus U through inner shaft 1108, nozzle 1112, and one or more holes 1114. Exhaust system 1118 is fluidly coupled to outer shaft 1106 and is configured to exhaust the thermal transfer medium from uterus U through outer shaft 1106.

Instrument 1100 further includes first tube 1150A and second tube 1150B extending through outer shaft 1106 in the embodiment shown in FIGS. 15A-15B. In alternate embodiments, instrument 1100 can include a single tube. In alternate embodiments, first tube 1150A and second tube 1150B can extend through inner shaft 1108 and nozzle 1112. As shown in FIG. 15A, first tube 1150A and second tube 1150B are fluidly coupled to vacuum source 1152. As further shown in FIG. 15A, when instrument 1100 is in a stowed position, nozzle 1112, first tube 1150A, and second tube 1150B are positioned in outer shaft 1106. When a distal end of outer shaft 1106 is positioned in uterus U and instrument 1100 is deployed, nozzle 1112, first tube 1150A, and second tube 1150B are moved out of outer shaft 1106. As first tube 1150A and second tube 1150B are moved out of outer shaft 1106, first tube 1150A and second tube 1150B will move outwards. A distal end of first tube 1150A will be positioned at an opening to first fallopian tube F1, and a distal end of second tube 1150B will be positioned at an opening to second fallopian tube F2. As a thermal transfer medium flows into uterus U through one or more holes 1114 in nozzle 1112, vacuum source 1152 can pull a vacuum through first tube 1150A and second tube 1150B to close first fallopian tube F1 and second fallopian tube F2, as shown in FIG. 15B, to prevent the thermal transfer medium from flowing through first fallopian tube F1 and second fallopian tube F2.

First tube 1150A and second tube 1150B can move outwards towards first fallopian tube F1 and second fallopian tube F2 using any suitable mechanism. For example, first tube 1150A and second tube 1150B can spring outwards. By further example, first tube 1150A and second tube 1150B can be guided outwards with guide wires. Additionally, first tube 1150A and second tube 1150B can include echogenic materials so that ultrasound can be used to guide the placement of first tube 1150A and second tube 1150B.

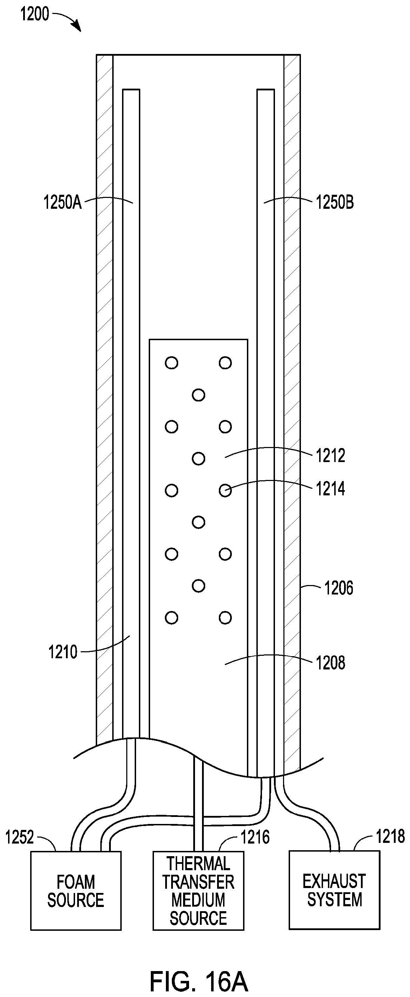

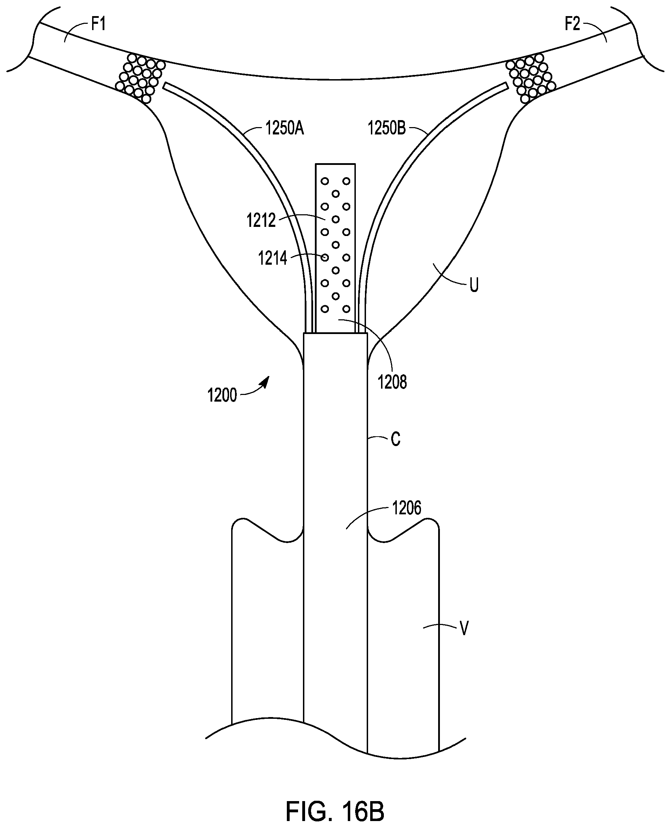

FIG. 16A is a schematic view of instrument 1200, foam source 1252, thermal transfer medium source 1216, and exhaust system 1218. FIG. 16B is a schematic view of a distal end of instrument 1200 positioned in uterus U. Instrument 1200 includes outer shaft 1206, inner shaft 1208, gap 1210, nozzle 1212, and one or more holes 1214. FIG. 16A also shows thermal transfer medium source 1216 and exhaust system 1218. FIGS. 16A-16B further show first tube 1250A and second tube 1250B of instrument 1200, and FIG. 16A shows foam source 1252. FIG. 16B also shows vagina V, cervix C, uterus U, and first fallopian tube F1 and second fallopian tube F2.

Instrument 1200 has generally the same structure and design as instrument 100 described above in reference to FIGS. 1A-1C, however instrument 1200 also includes first tube 1250A, second tube 1250B, and foam source 1252. First tube 1250A and second tube 1250B and foam source 1252 are described here with respect to instrument 1200, but can also be included on any of the embodiments of an instrument descried herewith, including instruments 100, 200, 300, 400, 500, 600, 700, 800, 900, 1000, 1100, 1300, 1400, 1500, and 1600.

Instrument 1200 can include a hand piece and a trigger, not shown in FIGS. 16A-16B. Outer shaft 1206 of instrument 1200 is configured to be inserted through vagina V and cervix C and into uterus U. Inner shaft 1208 is configured to be deployed from outer shaft 1206 when a distal end of outer shaft 1206 is positioned in uterus U. Gap 1210 is formed between an outer surface of inner shaft 1208 and an inner surface of outer shaft 1206. Nozzle 1212 forms a distal end of inner shaft 1208 and includes one or more holes 1214 that are configured to deliver a thermal transfer medium to uterus U. Any suitable thermal transfer medium, such as those discussed above in reference to FIGS. 2-5, can be delivered to uterus U.

Thermal transfer medium source 1216 is fluidly coupled to inner shaft 1208 and is configured to deliver a thermal transfer medium to uterus U through inner shaft 1208, nozzle 1212, and one or more holes 1214. Exhaust system 1218 is fluidly coupled to outer shaft 1206 and is configured to exhaust the thermal transfer medium from uterus U through outer shaft 1206.

Instrument 1200 further includes first tube 1250A and second tube 1250B extending through outer shaft 1206 in the embodiment shown in FIGS. 16A-16B. In alternate embodiments, instrument 1200 can include a single tube. In alternate embodiments, first tube 1250A and second tube 1250B can extend through inner shaft 1208 and nozzle 1212. As shown in FIG. 16A, first tube 1250A and second tube 1250B are fluidly coupled to vacuum source 1252. As further shown in FIG. 16A, when instrument 1200 is in a stowed position, nozzle 1212, first tube 1250A, and second tube 1250B are positioned in outer shaft 1206. When a distal end of outer shaft 1206 is positioned in uterus U and instrument 1200 is deployed, nozzle 1212, first tube 1250A, and second tube 1250B are moved out of outer shaft 1206. As first tube 1250A and second tube 1250B are moved out of outer shaft 1206, first tube 1250A and second tube 1250B will move outwards. A distal end of first tube 1250A will be positioned at an opening to first fallopian tube F1, and a distal end of second tube 1250B will be positioned at an opening to second fallopian tube F2. A foam from foam source 1252 can be dispensed through first tube 1250A and second tube 1250B to the openings of first fallopian tube F1 and second fallopian tube F2 to form a foam blockage at the openings of first fallopian tube F1 and second fallopian tube F2, as shown in FIG. 16B. As a thermal transfer medium flows into uterus U through one or more holes 1214 in nozzle 1212, the foam blockage will prevent the thermal transfer medium from flowing through first fallopian tube F1 and second fallopian tube F2.

First tube 1250A and second tube 1250B can move outwards towards first fallopian tube F1 and second fallopian tube F2 using any suitable mechanism. For example, first tube 1250A and second tube 1250B can spring outwards. By further example, first tube 1250A and second tube 1250B can be guided outwards with guide wires. Additionally, first tube 1250A and second tube 1250B can include echogenic materials so that ultrasound can be used to guide the placement of first tube 1250A and second tube 1250B.

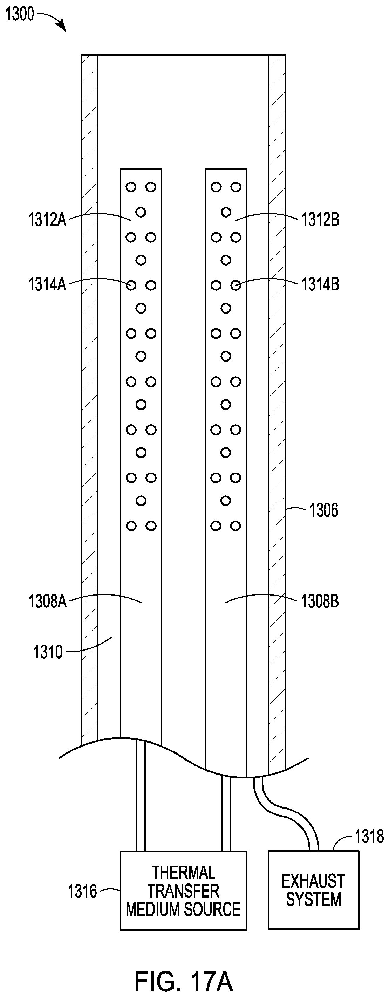

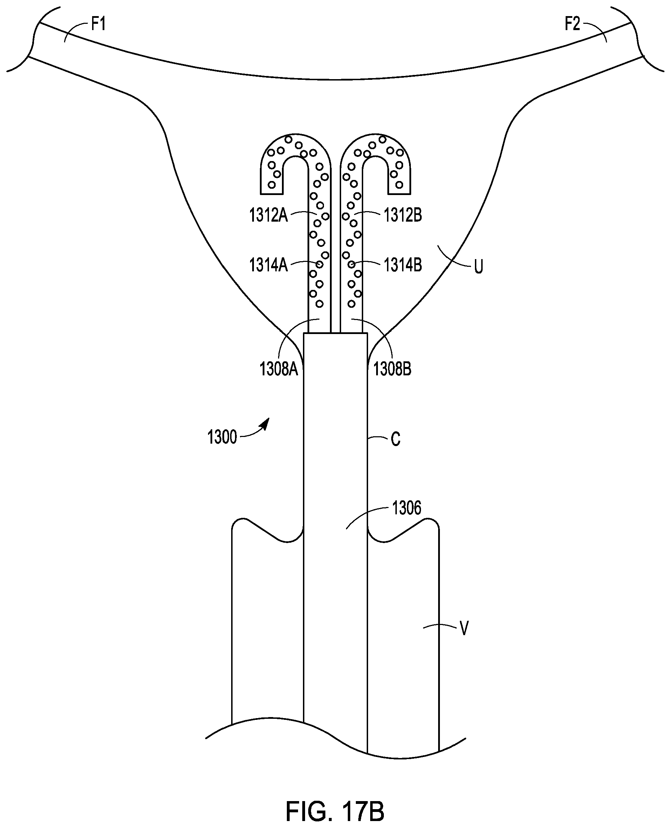

FIG. 17A is a schematic view of instrument 1300, thermal transfer medium source 1316, and exhaust system 1318. FIG. 17B is a schematic view of a distal end of instrument 1300 positioned in uterus U. Instrument 1300 includes outer shaft 1306, first inner shaft 1308A, second inner shaft 1308B, gap 1310, first nozzle 1312A, second nozzle 1312B, first one or more holes 1314A, and second one or more holes 1314B. FIG. 17A also shows thermal transfer medium source 1316 and exhaust system 1318. FIG. 17B also shows vagina V, cervix C, uterus U, and first fallopian tube F1 and second fallopian tube F2.

Instrument 1300 has generally the same structure and design as instrument 100 described above in reference to FIGS. 1A-1C, however instrument 1300 includes two inner shafts, including first inner shaft 1308A, two nozzles, including first nozzle 1312A and second nozzle 1312B, and two pluralities of holes, including first one or more holes 1314A and second one or more holes 1314B. First inner shaft 1308A, second inner shaft 1308B, first nozzle 1312A, second nozzle 1312B, first one or more holes 1314A, and second one or more holes 1314B are described here with respect to instrument 1300, but can also be included on any of the embodiments of an instrument descried herewith, including instruments 100, 200, 300, 400, 500, 600, 700, 800, 900, 1000, 1100, 1200, 1400, 1500, and 1600.

Instrument 1300 can include a hand piece and a trigger, not shown in FIGS. 17A-17B. Outer shaft 1306 of instrument 1300 is configured to be inserted through vagina V and cervix C and into uterus U. Instrument 1300 includes first inner shaft 1308A and second inner shaft 1308B positioned next to one another extending through outer shaft 1300. First inner shaft 1308A and second inner shaft 1308B are configured to be deployed from outer shaft 1306 when a distal end of outer shaft 1306 is positioned in uterus U. Gap 1310 is formed between outer surfaces of first inner shaft 1308A and second inner shaft 1308B and an inner surface of outer shaft 1306. First nozzle 1312A is formed at a distal end of first inner shaft 1308A, and second nozzle 1312B is formed at a distal end of second inner shaft 1308B. First nozzle 1312A includes first one or more holes 1314A, and second nozzle 1312B includes second one or more holes 1314B. First one or more holes 1314A and second one or more holes 1314B are configured to deliver a thermal transfer medium to uterus U. Any suitable thermal transfer medium, such as those discussed above in reference to FIGS. 2-5, can be delivered to uterus U.

Thermal transfer medium source 1316 is fluidly coupled to first inner shaft 1308A and second inner shaft 1308B and is configured to deliver a thermal transfer medium to uterus U through first inner shaft 1308A, second inner shaft 1308B, first nozzle 1312A, second nozzle 1312B, first one or more holes 1314A, and second one or more holes 1314B. In the embodiment shown in FIGS. 17A-17B, thermal transfer medium source 1316 is separately fluidly coupled to each of first inner shaft 1308A and second inner shaft 1308B. In alternate embodiments, first inner shaft 1308A and second inner shaft 1308B can have a shared proximal end that is fluidly coupled to thermal transfer medium source 1316. In an alternate embodiment, first inner shaft 1308A and second inner shaft 1308B can be connected to one another at a proximal end. Exhaust system 1318 is fluidly coupled to outer shaft 1306 and is configured to exhaust the thermal transfer medium from uterus U through outer shaft 1306.

As shown in FIG. 17B, first inner shaft 1308A and second inner shaft 1308B are shaped as hooks when deployed in uterus U. The shape of first inner shaft 1308A and second inner shaft 1308B can be configured to control the flow of the thermal transfer medium out through pluralities of holes 1314 and onto particular areas of uterus U. As shown in FIG. 17A, first inner shaft 1308A and second inner shaft 1308B will be held straight when they are stowed in outer shaft 1306. When first inner shaft 1308A and second inner shaft 1308B are deployed from outer shaft 1306, first inner shaft 1308A and second inner shaft 1308B can assume the shape of hooks using any suitable mechanism. For example, first inner shaft 1308A and second inner shaft 1308B can include a shape memory alloy that will cause first inner shaft 1308A and second inner shaft 1308B to assume the shape of hooks. In a further example, first inner shaft 1308A and second inner shaft 1308B can be guided to the shape of hooks using guide wires.

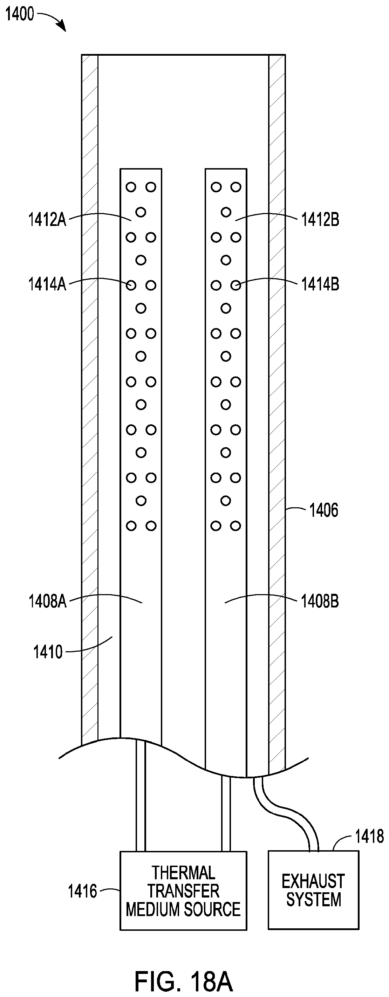

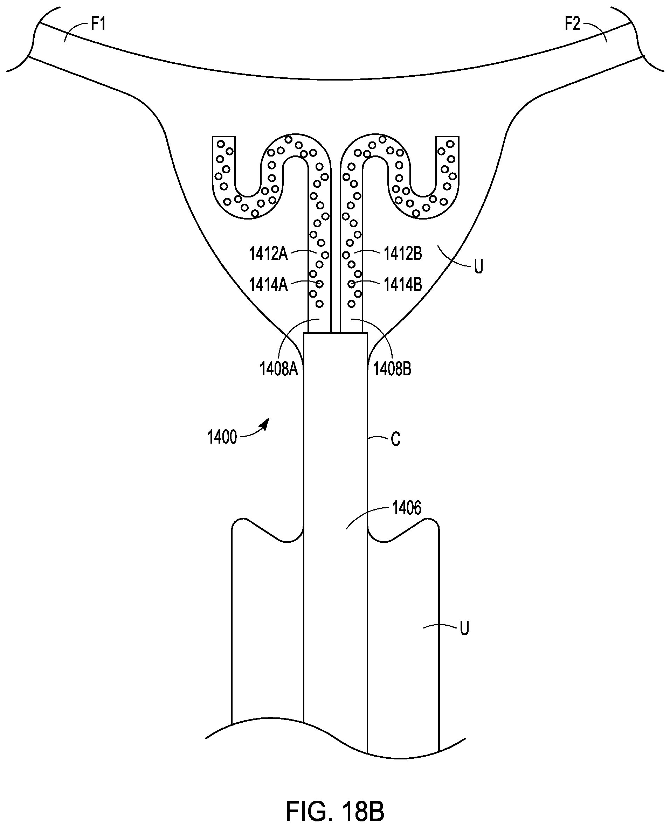

FIG. 18A is a schematic view of instrument 1400, thermal transfer medium source 1416, and exhaust system 1418. FIG. 18B is a schematic view of a distal end of instrument 1400 positioned in uterus U. Instrument 1400 includes outer shaft 1406, first inner shaft 1408 A, second inner shaft 1408B, gap 1410, first nozzle 1412A, second nozzle 1412B, first one or more holes 1414A, and second one or more holes 1414B. FIG. 18A also shows thermal transfer medium source 1416 and exhaust system 1418. FIG. 18B also shows vagina V, cervix C, uterus U, and first fallopian tube F1 and second fallopian tube F2.

Instrument 1400 has generally the same structure and design as instrument 100 described above in reference to FIGS. 1A-1C, however instrument 1400 includes two inner shafts, including first inner shaft 1408 A and second inner shaft 1408B, two nozzles, including first nozzle 1412A and second nozzle 1412B, and two pluralities of holes, including first one or more holes 1414A and second one or more holes 1414B. First inner shaft 1408 A, second inner shaft 1408B, gap 1410, first nozzle 1412A, second nozzle 1412B, first one or more holes 1414A, and second one or more holes 1414B are described here with respect to instrument 1400, but can also be included on any of the embodiments of an instrument descried herewith, including instruments 100, 200, 300, 400, 500, 600, 700, 800, 900, 1000, 1100, 1200, 1300, 1500, and 1600.

Instrument 1400 can include a hand piece and a trigger, not shown in FIGS. 18A-18B. Outer shaft 1406 of instrument 1400 is configured to be inserted through vagina V and cervix C and into uterus U. Instrument 14300 includes two first inner shaft 1408A and second inner shaft 1408B positioned next to one another extending through outer shaft 1400. First inner shaft 1408A and second inner shaft 1408B are configured to be deployed from outer shaft 1406 when a distal end of outer shaft 1406 is positioned in uterus U. Gap 1410 is formed between outer surfaces of first inner shaft 1408A and second inner shaft 1408B and an inner surface of outer shaft 1406. First nozzle 1412A is formed at a distal end of first inner shaft 1408A, and second nozzle 1412B is formed at a distal end of second inner shaft 1408B. First nozzle 1412A includes first one or more holes 1414A, and second nozzle 1412B includes second one or more holes 1414B. First one or more holes 1414A and second one or more holes 1414B are configured to deliver a thermal transfer medium to uterus U. Any suitable thermal transfer medium, such as those discussed above in reference to FIGS. 2-5, can be delivered to uterus U.

Thermal transfer medium source 1416 is fluidly coupled to first inner shaft 1408A and second inner shaft 1408B and is configured to deliver a thermal transfer medium to uterus U through first inner shaft 1408 A, second inner shaft 1408B, gap 1410, first nozzle 1412A, second nozzle 1412B, first one or more holes 1414A, and second one or more holes 1414B. In the embodiment shown in FIGS. 18A-18B, thermal transfer medium source 1416 is separately fluidly coupled to each of first inner shaft 1408A and second inner shaft 1408B. In alternate embodiments, first inner shaft 1408A and second inner shaft 1408B have a shared proximal end that is fluidly coupled to thermal transfer medium source 1416. In an alternate embodiment, first inner shaft 1408A and second inner shaft 1408B can be connected to one another at a proximal end. Exhaust system 1418 is fluidly coupled to outer shaft 1406 and is configured to exhaust the thermal transfer medium from uterus U through outer shaft 1406.

As shown in FIG. 18B, first inner shaft 1408A and second inner shaft 1408B are shaped as waves when deployed in uterus U. The shape of first inner shaft 1408A and second inner shaft 1408B can be configured to control the flow of the thermal transfer medium out through first one or more holes 1414A and second one or more holes 1414B and onto particular areas of uterus U. As shown in FIG. 18A, first inner shaft 1408A and second inner shaft 1408B will be held straight when they are stowed in outer shaft 1406. When first inner shaft 1408A and second inner shaft 1408B are deployed from outer shaft 1406, first inner shaft 1408A and second inner shaft 1408B can assume the shape of waves using any suitable mechanism. For example, first inner shaft 1408A and second inner shaft 1408B can include a shape memory alloy that will cause first inner shaft 1408A and second inner shaft 1408B to assume the shape of waves. In a further example, first inner shaft 1408A and second inner shaft 1408B can be guided to the shape of waves using guide wires.

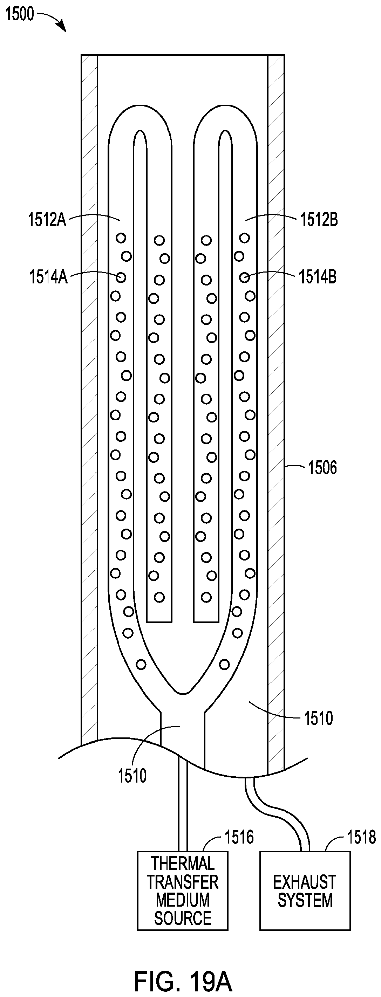

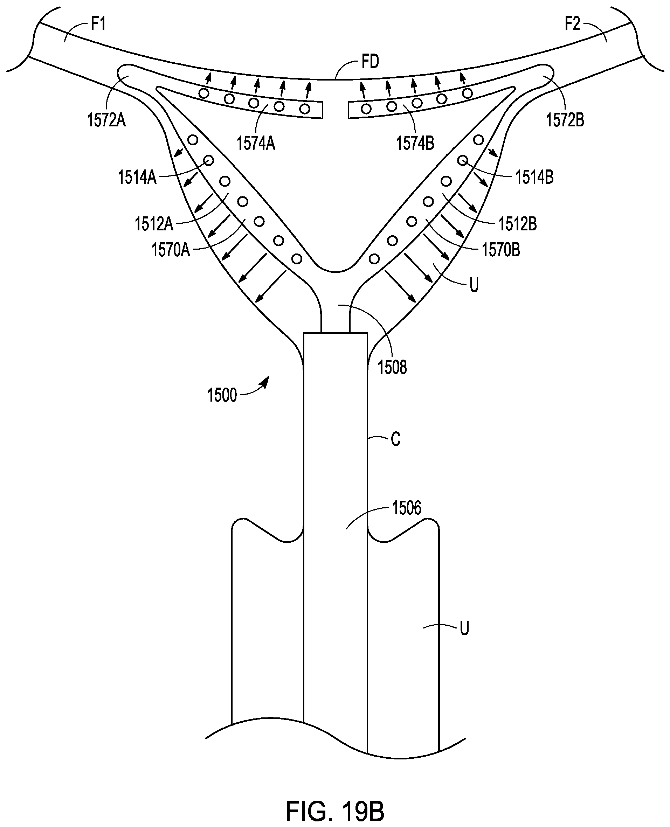

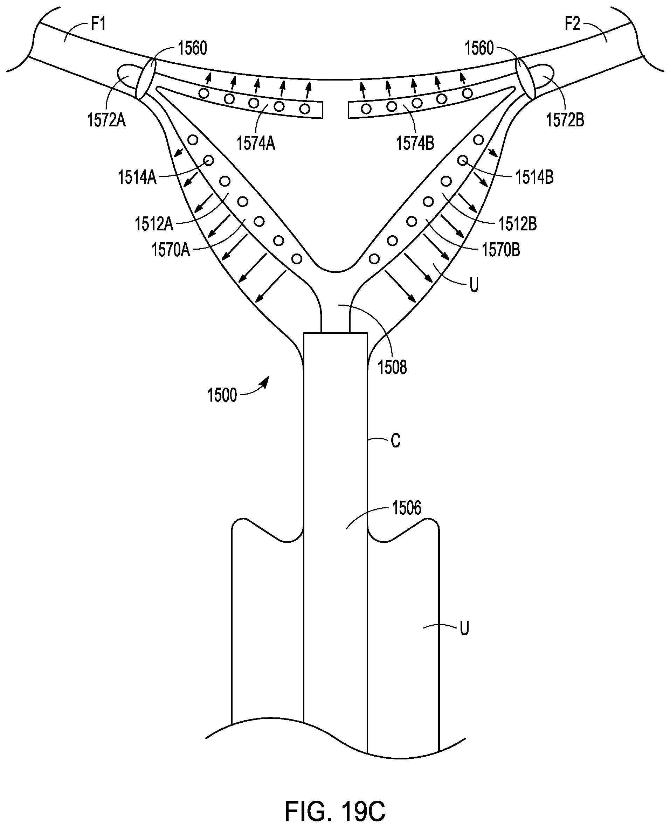

FIG. 19A is a schematic view of instrument 1500, thermal transfer medium source 1516, and exhaust system 1518. FIG. 19B is a schematic view of a distal end of instrument 1500 positioned in uterus U. FIG. 19C is a schematic view of the distal end of instrument 1500 that includes seals 1560 positioned in uterus U. Instrument 1500 includes outer shaft 1506, inner shaft 1508, gap 1510, first nozzle 1512A, second nozzle 1512B, first one or more holes 1514A, and second one or more holes 1514B. FIG. 19A also shows thermal transfer medium source 1516 and exhaust system 1518. FIG. 19C also shows first seal 1560A and second seal 1560B of instrument 1500. FIGS. 15B-15C also shows vagina V, cervix C, uterus U, first fallopian tube F1 and second fallopian tube F2, and fundus FD.

Instrument 1500 has generally the same structure and design as instrument 100 described above in reference to FIGS. 1A-1C, however instrument 1500 includes two nozzles, including first nozzle 1512A and second nozzle 1512B, two pluralities of holes, including first one or more holes 1514A, and second one or more holes 1514B, and also includes first seal 1560A and second seal 1560B. First nozzle 1512A, second nozzle 1512B, first one or more holes 1514A, second one or more holes 1514B, first seal 1560A, and second seal 1560B are described here with respect to instrument 1500, but can also be included on any of the embodiments of an instrument descried herewith, including instruments 100, 200, 300, 400, 500, 600, 700, 800, 900, 1000, 1100, 1200, 1300, 1400, and 1600.

Instrument 1500 can include a hand piece and a trigger, not shown in FIGS. 19A-19C. Outer shaft 1506 of instrument 1500 is configured to be inserted through vagina V and cervix C and into uterus U. Inner shaft 1508 is configured to be deployed from outer shaft 1506 when a distal end of outer shaft 1506 is positioned in uterus U. Gap 1510 is formed between an outer surface of inner shaft 1508 and an inner surface of outer shaft 1506. Inner shaft 1508 splits into first nozzle 1512A and second nozzle 1512B at distal end of inner shaft 1508. First nozzle 1512A includes first one or more holes 1514A, and second nozzle 1512B includes second one or more holes 1514B. First one or more holes 1514A and second one or more holes 1514B are configured to deliver a thermal transfer medium to uterus U. Any suitable thermal transfer medium, such as those discussed above in reference to FIGS. 2-5, can be delivered to uterus U.

Thermal transfer medium source 1516 is fluidly coupled to inner shaft 1508 and is configured to deliver a thermal transfer medium to uterus U through inner shaft 1508, first nozzle 1512A, second nozzle 1512B, first one or more holes 1514A, and second one or more holes 1514B. Exhaust system 1518 is fluidly coupled to outer shaft 1506 and is configured to exhaust the thermal transfer medium from uterus U through outer shaft 1506.

As shown in FIG. 19B, first nozzle 1512A is shaped to have first portion 1570A that extends along a side of uterus U, bend portion 1572A that is positioned at an opening to one fallopian tube F, and second portion 1574A that extends along fundus FD of uterus U. Second nozzle 1512B is shaped to have first portion 1570B that extends along a side of uterus U, bend portion 1572B that is positioned at an opening to one fallopian tube F, and second portion 1574B that extends along fundus FD of uterus U. First nozzle 1512A and second nozzle 1512B are shaped to direct the flow of thermal transfer medium onto the sides and fundus FD of uterus U. First one or more holes 1514A on first nozzle 1512A are positioned on first portion 1570A and second portion 1570A of first nozzle 1512A to direct the thermal transfer medium to the first wall and fundus FD of uterus U. Second one or more holes 1514B on second nozzle 1512B are positioned on first portion 1570B and second portion 1574B of second nozzle 1512B to direct the thermal transfer medium to the first wall and fundus FD of uterus U. As shown in FIG. 19B, first nozzle 1512A and second nozzle 1512B will be held in a bent configuration in outer shaft 1506. When inner shaft 1508 is deployed from outer shaft 1506, first nozzle 1512A and second nozzle 1512B can assume the shape extending along a side of uterus U and along fundus FD of uterus U using any suitable mechanism. For example, first nozzle 1512A and second nozzle 1512B can include a shape memory alloy that will cause first nozzle 1512A and second nozzle 1512B to assume the shape. In a further example, first nozzle 1512A and second nozzle 1512B can be guided to the shape using guide wires.

Instrument 1500 further includes first seal 1560A and second seal 1560B. First seal 1560A is positioned at bend portion 1572A of first nozzle 1512A at the opening to first fallopian tube F1. Second seal 1560B is positioned at bend portion 1572B of second nozzle 1512B at the opening to second fallopian tube F2. First seal 1560A and second seal 1560B can be fluidly coupled to first nozzle 1512A and second nozzle 1512B. As a thermal transfer medium is delivered to uterus U through first nozzle 1512A and second nozzle 1512B, the thermal transfer medium can flow into first seal 1560A and second seal 1560B. First seal 1560A and second seal 1560B will expand and form a seal with first fallopian tube F1 and second fallopian tube F2. When expanded, first seal 1560A and second seal 1560B will prevent thermal transfer medium in uterus U from flowing through first fallopian tube F1 and second fallopian tube F2.

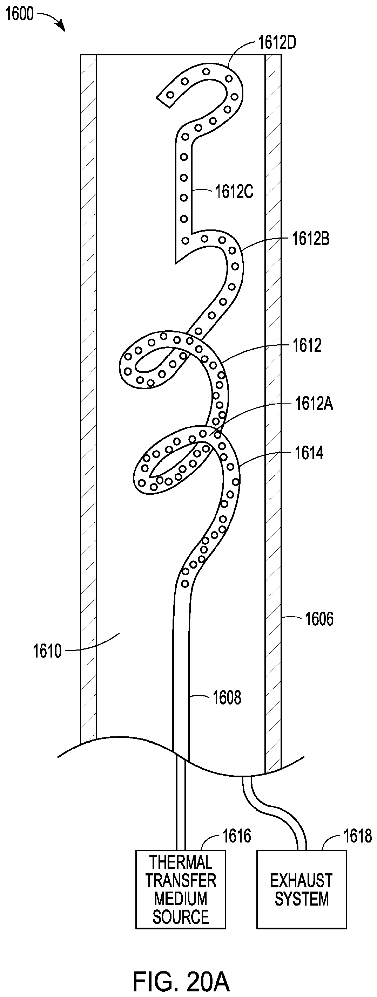

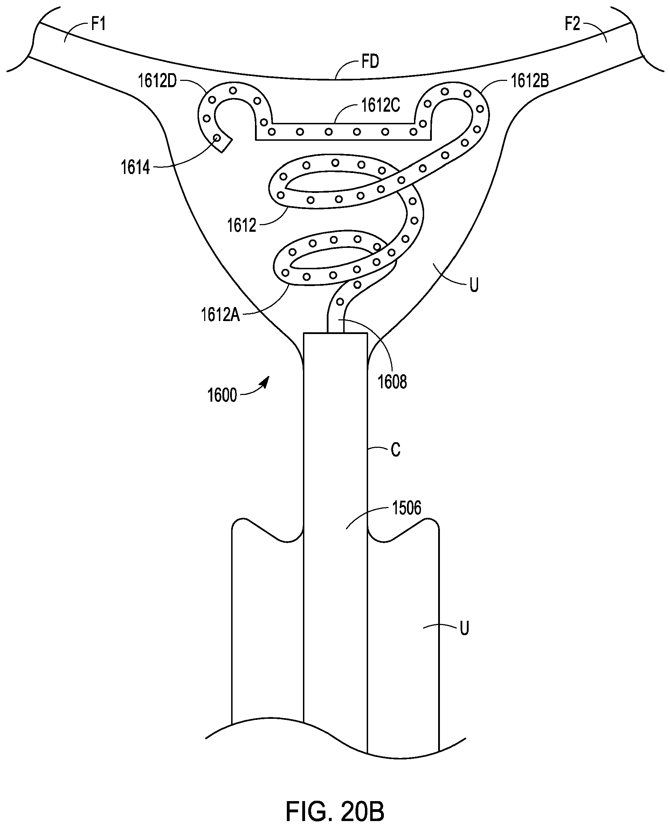

FIG. 20A is a schematic view of instrument 1600, thermal transfer medium source 1616, and exhaust system 1618. FIG. 20B is a schematic view of a distal end of instrument 1600 positioned in uterus U. Instrument 1600 includes outer shaft 1606, inner shaft 1608, gap 1610, nozzle 1612, and one or more holes 1614. FIG. 20A also shows thermal transfer medium source 1616 and exhaust system 1618. FIG. 20B also shows vagina V, cervix C, uterus U, first fallopian tube F1 and second fallopian tube F2, and fundus FD.

Instrument 1600 has generally the same structure and design as instrument 100 described above in reference to FIGS. 1A-1C, however instrument 300 also includes nozzle 1612 having a curled shape. Nozzle 1612 is described here with respect to instrument 1600, but can also be included on any of the embodiments of an instrument descried herewith, including instruments 100, 200, 300, 400, 500, 600, 700, 800, 900, 1000, 1100, 1200, 1300, 1400, and 1500.

Instrument 1600 can include a hand piece and a trigger, not shown in FIGS. 20A-20B. Outer shaft 1606 of instrument 1600 is configured to be inserted through vagina V and cervix C and into uterus U. Inner shaft 1608 is configured to be deployed from outer shaft 1606 when a distal end of outer shaft 1606 is positioned in uterus U. Gap 1610 is formed between an outer surface of inner shaft 1608 and an inner surface of outer shaft 1606. Nozzle 1612 forms a distal end of inner shaft 1608 and includes one or more holes 1614 that are configured to deliver a thermal transfer medium to uterus U. Any suitable thermal transfer medium, such as those discussed above in reference to FIGS. 2-5, can be delivered to uterus U.

Thermal transfer medium source 1616 is fluidly coupled to inner shaft 1608 and is configured to deliver a thermal transfer medium to uterus U through inner shaft 1608, nozzle 1612, and one or more holes 1614. Exhaust system 1618 is fluidly coupled to outer shaft 1606 and is configured to exhaust the thermal transfer medium from uterus U through outer shaft 1606.

As shown in FIG. 20B, nozzle 1612 has a curled shape when it is deployed in uterus U. Nozzle 1612 has first portion 1612A that swirls around twice through a center portion of uterus U, second portion 1612B that is curled towards an opening to a first fallopian tube F, third portion 1612C that extends along fundus FD of uterus U, and fourth portion 1612D that is curled towards an opening to a second fallopian tube F. In alternate embodiments, nozzle 1612 can have any curled shape. One or more holes 1614 are positioned on first portion 1612A of nozzle 1612 to direct the thermal transfer medium towards the walls of uterus U. One or more holes 1614 are positioned on second portion 1612B and fourth portion 1612D of nozzle 1612 to direct the thermal transfer medium towards the walls and fundus FD of uterus U but not towards first fallopian tube F1 and second fallopian tube F2. One or more holes 1614 are positioned on third portion 1612C of nozzle 1612 to direct the thermal transfer medium towards fundus FD of uterus U.

## Claims

1. An instrument for producing a tissue effect at or near a uterine wall, the instrument comprising:
an outer shaft (1506) that is configured to extend through a vagina and a cervix and into a uterus; and
an inner shaft (1508) positioned in the outer shaft, wherein a distal portion of the inner shaft (1508) is configured for receiving a thermal transfer medium,
the distal portion including a first nozzle (1512A) and a second nozzle (1512B), wherein each nozzle includes:
a first portion (1570A, 1570B) configured to extend along a side of the uterus, the first portion (1570A, 1570B) including a first plurality of holes (1514A, 1514B);
a second portion (1574A, 1574B) configured to extend along a fundus of the uterus, the second portion (1574A, 1574B) including a second plurality of holes (1514A, 1514B); and
a bend portion (1572A, 15712B) positioned between the first and the second portions, the bend portion (1572A, 15712B) configured to be positioned at an opening of a corresponding fallopian tube,
wherein the distal portion is configured to be in fluid communication with at least a portion of a target treatment site, the target treatment site being at or near the uterine wall,
such that the distal portion delivers the thermal transfer medium toward the target treatment site, and thereby produce the tissue effect at or near the uterine wall;
wherein each nozzle further includes:
a seal (1560A, 1560B) positioned at the bend portion, the seal configured to prevent thermal transfer medium in uterus from flowing through first fallopian tube and second fallopian tube;
wherein the bend portion (1572A, 1572B) of each of the first and second nozzle (1512A, 1512B) includes the seal (1560A, 1560B), wherein the seal (1560A, 1560B) is configured to transition between an unexpanded state and an expanded state to block the opening of the corresponding fallopian tube;
**characterized in that** the seal (1560A, 1560B) is in fluid communication with the nozzle (1512A, 1512B) and is configured to transition to the expanded state when the nozzle (1512A, 1512B) receives the thermal transfer medium.

2. The instrument of claim 1, wherein the inner shaft (1508) is configured to slide within the outer shaft (1506) to move the distal end from an undeployed position to a deployed position.

3. The instrument of claim 1 or 2, further including:
a sealing portion (230) positioned around the outer shaft (1506) that is configured to form a seal between the outer shaft (1506) and the cervix when it is expanded;

4. The instrument of any of claims 1 to 3, further including:
a pressure regulator (1054) positioned on the outer shaft (1506) that is configured to control an inflow rate and an outflow rate of a thermal transfer medium to the uterus.

5. The instrument of any of claims 1 to 4, further including:
a temperature sensor (234) positioned on the outer shaft (1506) that is configured to sense a temperature of the uterus.

6. The instrument of any of claims 1 to 5, further including:
a pressure sensor (236) positioned on the outer shaft (1506) that is configured to sense a pressure of the uterus.

7. The instrument of any of claims 1 to 6, and further including:
an exhaust system (1518) fluidly coupled to the outer shaft (1506).

8. The instrument of any of claims 1 to 7, wherein the first nozzle (1512A) and the second nozzle (1512B) include a shape memory alloy.

9. A system for ablating an endometrium of a uterus, the system comprising:
the instrument of any of claims 1 to 8, and
a thermal transfer medium source (1516) in fluid communication with the distal portion of the inner shaft (1508) of the instrument.

10. The system of claim 9, wherein the thermal transfer medium is in a cryogenic state.

11. The system of claim 9 or 10, wherein the thermal transfer medium is a cryogenic supercritical fluid.

12. The system of any of claims 9 to 11, wherein the thermal transfer medium includes a transport medium and a plurality of thermal transfer particles that are delivered to the uterus.

13. The system of claim 12, wherein the plurality of thermal transfer particles are configured to contact the endometrial lining of the uterus.

## Patentansprüche

1. Ein Instrument zur Erzeugung eines Gewebeeffekts an oder nahe einer Gebärmutterwand, wobei das Instrument umfasst:
einen äußeren Schaft (1506), der dazu eingerichtet ist, sich durch die Vagina und den Gebärmutterhals in die Gebärmutter zu erstrecken; und
einen inneren Schaft (1508), der in dem äußeren Schaft positioniert ist, wobei ein distaler Abschnitt des inneren Schafts (1508) dazu eingerichtet ist, ein Wärmeübertragungsmedium aufzunehmen,
wobei der distale Abschnitt eine erste Düse (1512A) und eine zweite Düse (1512B) umfasst, wobei jede Düse umfasst:
einen ersten Abschnitt (1570A, 1570B), der dazu eingerichtet ist, dass er sich entlang einer Seite der Gebärmutter erstreckt, wobei der erste Abschnitt (1570A, 1570B) eine erste Mehrzahl von Löchern (1514A, 1514B) umfasst;
einen zweiten Abschnitt (1574A, 1574B), der dazu eingerichtet ist, dass er sich entlang eines Fundus des Uterus erstreckt, wobei der zweite Abschnitt (1574A, 1574B) eine zweite Mehrzahl von Löchern (1514A, 1514B) umfasst; und
einen Biegeabschnitt (1572A, 15712B), der zwischen dem ersten und dem zweiten Abschnitt positioniert ist, wobei der Biegeabschnitt (1572A, 15712B) dazu eingerichtet ist, dass er an einer Öffnung eines entsprechenden Eileiters positioniert ist,
wobei der distale Abschnitt dazu eingerichtet ist, dass er in Fluidverbindung mit mindestens einem Abschnitt eines Zielgebiets steht, wobei sich das Zielgebiet an oder in der Nähe der Gebärmutterwand befindet, so dass der distale Abschnitt das Wärmeübertragungsmedium in Richtung des Zielgebiets abgibt und dadurch den Gewebeeffekt an oder in der Nähe der Gebärmutterwand erzeugt;
wobei jede Düse ferner umfasst:
eine Dichtung (1560A, 1560B), die am gebogenen Abschnitt positioniert ist, wobei die Dichtung dazu eingerichtet ist, dass sie verhindert, dass das Wärmeübertragungsmedium in der Gebärmutter durch den ersten Eileiter und den zweiten Eileiter fließt;
wobei der gebogene Abschnitt (1572A, 1572B) jeder der ersten und zweiten Düse (1512A, 1512B) die Dichtung (1560A, 1560B) umfasst, wobei die Dichtung (1560A, 1560B) dazu eingerichtet ist, zwischen einem nicht expandierten Zustand und einem expandierten Zustand zu wechseln, um die Öffnung des entsprechenden Eileiters zu blockieren;
**dadurch gekennzeichnet, dass** die Dichtung (1560A, 1560B) in Fluidverbindung mit der Düse (1512A, 1512B) steht und dazu eingerichtet ist, in den expandierten Zustand überzugehen, wenn die Düse (1512A, 1512B) das thermische Übertragungsmedium empfängt.

2. Das Instrument nach Anspruch 1, wobei der innere Schaft (1508) dazu eingerichtet ist, innerhalb des äußeren Schafts (1506) zu gleiten, um das distale Ende aus einer nicht entfalteten Position in eine entfaltete Position zu bewegen.

3. Das Instrument nach Anspruch 1 oder 2, das ferner umfasst:
einen Dichtungsabschnitt (230), der um den äußeren Schaft (1506) herum angeordnet ist und dazu eingerichtet ist, eine Dichtung zwischen dem äußeren Schaft (1506) und dem Gebärmutterhals zu bilden, wenn er expandiert ist.

4. Das Instrument gemäß einem der Ansprüche 1 bis 3, das ferner umfasst:
einen Druckregler (1054), der an dem äußeren Schaft (1506) positioniert ist und dazu eingerichtet ist, eine Zuflussrate und eine Abflussrate eines Wärmeübertragungsmediums zum Uterus zu steuern.

5. Das Instrument gemäß einem der Ansprüche 1 bis 4, das ferner umfasst:
einen Temperatursensor (234), der an dem äußeren Schaft (1506) positioniert ist und dazu eingerichtet ist, eine Temperatur der Gebärmutter zu erfassen.

6. Das Instrument nach einem der Ansprüche 1 bis 5, das ferner umfasst:
einen Drucksensor (236), der an dem äußeren Schaft (1506) positioniert ist und dazu eingerichtet ist, einen Druck der Gebärmutter zu erfassen.

7. Das Instrument gemäß einem der Ansprüche 1 bis 6, das ferner umfasst:
ein Ablaufsystem (1518), das fluidisch mit dem äußeren Schaft (1506) gekoppelt ist.

8. Das Instrument gemäß einem der Ansprüche 1 bis 7, wobei die erste Düse (1512A) und die zweite Düse (1512B) eine Formgedächtnislegierung enthalten.

9. Ein System zur Ablation des Endometriums einer Gebärmutter, das System umfasst:
das Instrument aus einem der Ansprüche 1 bis 8 und
eine Wärmeübertragungsmediumquelle (1516), die in Fluidverbindung mit dem distalen Abschnitt des inneren Schafts (1508) des Instruments steht.

10. Das System nach Anspruch 9, wobei sich das thermische Übertragungsmedium in einem kryogenen Zustand befindet.

11. Das System nach Anspruch 9 oder 10, wobei das thermische Übertragungsmedium ein kryogenes überkritisches Fluid ist.

12. Das System gemäß einem der Ansprüche 9 bis 11, wobei das thermische Übertragungsmedium ein Transportmedium und eine Mehrzahl von thermischen Übertragungspartikeln umfasst, die in die Gebärmutter abgegeben werden.

13. Das System nach Anspruch 12, wobei die Mehrzahl von thermischen Übertragungspartikeln dazu eingerichtet ist, mit den Endometriumschichten der Gebärmutter in Kontakt zu kommen.

## Revendications

1. Instrument destiné à produire un effet tissulaire au niveau ou à proximité d'une paroi utérine, l'instrument comprenant:
un arbre externe (1506) configuré pour s'étendre à travers un vagin et un col de l'utérus et dans un utérus ; et
un arbre interne (1508) positionné dans l'arbre externe, dans lequel une partie distale de l'arbre interne (1508) est configurée pour recevoir un moyen de transfert thermique,
la partie distale comprenant une première buse (1512A) et une deuxième buse (1512B), dans laquelle chaque buse comprend:
une première partie (1570A, 1570B) configurée pour s'étendre le long d'un côté de l'utérus, la première partie (1570A, 1570B) comprenant une première pluralité de trous (1514A, 1514B);
une deuxième partie (1574A, 1574B) configurée pour s'étendre le long d'un fond de l'utérus, la deuxième partie (1574A, 1574B) comprenant une deuxième pluralité de trous (1514A, 1514B); et
une partie courbée (1572A, 15712B) positionnée entre les première et deuxième parties, la partie courbée (1572A, 15712B) étant configurée pour être positionnée au niveau d'une ouverture d'une trompe de Fallope correspondante,
dans laquelle la partie distale est configurée pour être en communication fluidique avec au moins une partie d'un site de traitement cible, le site de traitement cible étant au niveau ou à proximité de la paroi utérine,
de telle sorte que la partie distale délivre le moyen de transfert thermique vers le site de traitement cible, et produit ainsi l'effet tissulaire au niveau ou à proximité de la paroi utérine;
dans lequel chaque buse comprend en outre:
un joint (1560A, 1560B) positionné au niveau de la partie courbée, le joint étant configuré pour empêcher le moyen de transfert thermique dans l'utérus de s'écouler à travers la première trompe de Fallope et la deuxième trompe de Fallope;
dans lequel la partie courbée (1572A, 1572B) de chacune des première et deuxième buses (1512A, 1512B) comprend le joint (1560A, 1560B), dans lequel le joint (1560A, 1560B) est configuré pour passer d'un état non expansé à un état expansé afin de bloquer l'ouverture de la trompe de Fallope correspondante;
**caractérisé en ce que** le joint (1560A, 1560B) est en communication fluidique avec la buse (1512A, 1512B) et est configuré pour passer à l'état expansé lorsque la buse (1512A, 1512B) reçoit le moyen de transfert thermique.

2. Instrument selon la revendication 1, dans lequel l'arbre interne (1508) est configuré pour coulisser à l'intérieur de l'arbre externe (1506) afin de déplacer l'extrémité distale d'une position non déployée à une position déployée.

3. Instrument selon la revendication 1 ou 2, comprenant en outre:
une partie d'étanchéité (230) positionnée autour de l'arbre extérieur (1506) qui est configurée pour former un joint entre l'arbre extérieur (1506) et le col de l'utérus lorsqu'il est déployé.

4. Instrument de l'une quelconque des revendications 1 à 3, comprenant en outre:
un régulateur de pression (1054) positionné sur l'arbre externe (1506) qui est configuré pour contrôler un flux entrant et un flux sortant d'un moyen de transfert thermique vers l'utérus.

5. Instrument de l'une quelconque des revendications 1 à 4, comprenant en outre:
un senseur de température (234) positionné sur l'arbre externe (1506) qui est configuré pour détecter une température de l'utérus.

6. Instrument de l'une quelconque des revendications 1 à 5, comprenant en outre:
un capteur de pression (236) positionné sur l'arbre externe (1506) qui est configuré pour détecter une pression de l'utérus.

7. Instrument de l'une quelconque des revendications 1 à 6, comprenant en outre:
un système évacuant (1518) couplé de manière fluidique à l'arbre extérieur (1506).

8. Instrument selon l'une quelconque des revendications 1 à 7, dans lequel la première buse (1512A) et la deuxième buse (1512B) comprennent un alliage à mémoire de forme.

9. Système pour l'ablation de l'endomètre d'un utérus, le système comprenant:
l'instrument de l'une quelconque des revendications 1 à 8, et
une source de moyen de transfert thermique (1516) en communication fluidique avec la partie distale de l'arbre interne (1508) de l'instrument.

10. Système selon la revendication 9, dans lequel le moyen de transfert thermique est à l'état cryogénique.

11. Système selon la revendication 9 ou 10, dans lequel le moyen de transfert thermique est un fluide cryogénique supercritique.

12. Système selon l'une quelconque des revendications 9 à 11, dans lequel le moyen de transfert thermique comprend un moyen de transport et une pluralité de particules de transfert thermique qui sont délivrées à l'utérus.

13. Système selon la revendication 12, dans lequel la pluralité de particules à transfert thermique est configurée pour entrer en contact avec la muqueuse endométriale de l'utérus.
